# EUROPEAN PATENT APPLICATION

(11) **EP 1 459 692 A1**
(43) Date of publication of application: **22.09.2004**
(21) Application number: 04076458.1
(22) Date of filing: 22.10.1997
(51) Int. Cl.: A61B 18/00

(54) **Method and apparatus for treating intervertebral discs**

(30) Priority: 23.10.1996 US 29735 P; 23.10.1996 US 29600 P; 23.10.1996 US 29734 P; 23.10.1996 US 29602 P; 08.05.1997 US 45941 P; 08.05.1997 US 46001 P; 08.05.1997 US 46002; 28.05.1997 US 47841 P; 28.05.1997 US 47820 P; 28.05.1997 US 47818 P; 28.05.1997 US 47848 P; 24.06.1997 US 881525; 24.06.1997 US 881692 P; 24.06.1997 US 881527; 24.06.1997 US 881693; 24.06.1997 US 881694
(62) Divisional of application: 97912882.4
(71) Applicant: Oratec Interventions, Inc., Menlo Park, CA 94025 (US)
(72) Inventor: Sharkey, Hugh J., Redwood City, CA 94065 (US); Ashley, John, San Francisco, CA 94127 (US); Saal, Jeffrey, Portola Valley, CA 94028 (US); Sall, Joel, Portola Valley, CA 94028 (US); Le, Le T., San José, CA 95132 (US); Weissman, Lee, San José, CA 95134 (US)
(74) Representative: Illingworth-Law, William

(57) **Abstract**

Percutaneous methods and systems for treating an intervertebral disc are disclosed. An intervertebral disc treatment apparatus includes an introducer (12) and a catheter (14) having a distal end (28), a proximal end, a longitudinal axis, and an intradiscal section (16) at the distal end on which there is at least one functional element (29). The treatment proceeds by applying a force longitudinally to the proximal end of the catheter which is sufficient to advance the intradiscal section through the nucleus pulposus and around an inner wall of an annulus fibrosus, but which force is insufficient to puncture the annulus fibrosus. The functional element is positioned at a selected location of the intervertebral disc by advancing or retracting the catheter and optionally twisting the proximal end of the catheter. Then, the functional unit treats the intervertebral disc. Optionally, there is an additional step of aspirating and/or adding a substance to the intervertebral disc.

## Description

### REFERENCE TO CO-PENDING APPLICATIONS

This application is a continuation-in-part of: U.S. Ser. No. 60/029,735, Attorney Docket No. 17616-720, entitled "Method and Apparatus for Treating Intervertebral Discs," filed October 23, 1996, now pending; U.S. Ser. No. 60/029,600, Attorney Docket No. 17616-738, entitled "Method and Apparatus for Treating Intervertebral Discs," filed October 23, 1996, now pending; U.S. Ser. No. 60/029,734, Attorney Docket No. 17616-751, entitled "Method and Apparatus for Treating Intervertebral Discs," filed October 23, 1996, now pending; U.S. Ser. No. 60/029,602, Attorney Docket No. 17616-752, entitled "Method and Apparatus for Treating Intervertebral Discs," filed October 23, 1996, now pending; Attorney Docket No. ORAT-016 (A.k.a. 17616-776), entitled "Method and Apparatus for Treating Intervertebral Discs with Radio Frequency Heating," filed May 8, 1997, now pending; U.S. Ser. No. 60/046,001, Attorney Docket No. ORAT-015, entitled "Method and Apparatus for Treating Intervertebral Discs with Resistive Heating," filed May 8, 1997, now pending; U.S. Ser. No. 60/047,820, Attorney Docket No. ORAT-016, entitled "Method and Apparatus for Treating Intervertebral Discs with Electromagnetic Energy," filed May 28, 1997, now pending; U.S. Serial No. Unknown, Attorney Docket No. ORAT-015, (A.k.a. 17616-778), entitled "Method and Apparatus for Treating Intervertebral Discs with Thermal Energy," filed May 28, 1997, now pending; U.S. Serial No. Unknown, Attorney Docket No. ORAT-017 (A.k.a. 17616-779), entitled "Method and Apparatus for Delivering or Removing Material from the Interior of an Intervertebral Disc," filed May 8, 1997, now pending; U.S. Serial No. Unknown, Attorney Docket No. ORAT-018 (A.k.a. 17616-780), entitled "Method and Apparatus for Treating Annular Fissures in Intervertebral Discs," filed May 28, 1997, now pending; U. S. Ser. No. 60/047,848, Attorney Docket No. ORAT-020, entitled "Method and Apparatus for Treating Intervertebral Disc Degeneration," filed May 28, 1997, now pending; U.S. Ser. No. 08/881,525, Attorney Docket No. ORAT-015/01US, entitled "Method and Apparatus for Treating Intervertebral Discs with Thermal Energy," filed June 24, 1997, now pending; U.S. Ser. No. 08/881,692, Attorney Docket No. ORAT-016/01US, entitled "Method and Apparatus for Treating Intervertebral Discs with Electromagnetic Energy," filed June 24, 1997, now pending; U.S. Ser. No. 08/881,527, Attorney Docket No. ORAT-017/01US, entitled "Method and Apparatus for Delivering or Removing Material from the Interior of an Intervertebral Disc," filed June 24, 1997, now pending; U.S. Ser. No. 08/881,693, Attorney Docket No. ORAT-018/01US, entitled "Method and Apparatus for Treating Annular Fissures in Intervertebral Discs," filed June 24, 1997, now pending; and U.S. Ser. No. 08/881,694, Attorney Docket No. ORAT-020/01US, entitled "Method and Apparatus for Treating Intervertebral Disc Degeneration," filed June 24, 1997, now pending, the entire contents of all of which are hereby incorporated herein by reference as if fully set forth herein.

### BACKGROUND

### Field of the Invention

This invention relates to methods and apparatuses to treat intervertebral disc problems and/or for modifying intervertebral disc tissue. More particularly this invention relates to percutaneous techniques to avoid major surgical intervention. In one embodiment, annular fissures are treated by radio frequency (RF)heating of intervertebral disc tissue.

### Description of Related Art

Intervertebral disc abnormalities (e.g., morphologic) have a high incidence in the population and may result in pain and discomfort if they impinge on or irritate nerves. Disc abnormalities may be the result of trauma, repetitive use, metabolic disorders and the aging process and include such disorders but are not limited to degenerative discs (i) localized tears or fissures in the annulus fibrosus, (ii) localized disc herniations with contained or escaped extrusions, and (iii) chronic, circumferential bulging disc.

Disc fissures occur rather easily after structural degeneration (a part of the aging process that may be accelerated by trauma) of fibrous components of the annulus fibrosus. Sneezing, bending or just attrition can tear these degenerated annulus fibers, creating a fissure. The fissure may or may not be accompanied by extrusion of nucleus pulposus material into or beyond the annulus fibrosus. The fissure itself may be the sole morphological change, above and beyond generalized degenerative changes in the connective tissue of the disc. Even if there is no visible extrusion, biochemicals within the disc may still irritate surrounding structures. Disc fissures can be debilitatingly painful. Initial treatment is symptomatic, including bed rest, pain killers and muscle relaxants. More recently spinal fusion with cages has been performed when conservative treatment did not relieve the pain. The fissure may also be associated with a herniation of that portion of the annulus.

With a contained disc herniation, there are no free nucleus fragments in the spinal canal. Nevertheless, even a contained disc herniation is problematic because the outward protrusion can press on the spinal nerves or irritate other structures. In addition to nerve root compression, escaped nucleus pulposus contents may chemically irritate neural structures. Current treatment methods include reduction of pressure on the annulus by removing some of the interior nucleus pulposus material by percutaneous nuclectomy. However, complications include disc space infection, nerve root injury, hematoma formation, instability of the adjacent vertebrae and collapse of the disc from decrease in height.

Another disc problem occurs when the disc bulges outward circumferentially in all directions and not just in one location. Over time, the disc weakens and takes on a "roll" shape or circumferential bulge. Mechanical stiffness of the joint is reduced and the joint may become unstable. One vertebra may settle on top of another. This problem continues as the body ages, and accounts for shortened stature in old age. With the increasing life expectancy of the population, such degenerative disc disease and impairment of nerve function are becoming major public health problems. As the disc "roll" extends beyond the normal circumference, the disc height may be compromised, and foramina with nerve roots are compressed. In addition, osteophytes may form on the outer surface of the disc roll and further encroach on the spinal canal and foramina through which nerves pass. This condition is called lumbar spondylosis.

It has been thought that such disc degeneration creates segmental instability which disturbs sensitive structures which in turn register pain. Traditional, conservative methods of treatment include bed rest, pain medication, physical therapy or steroid injection. Upon failure of conservative therapy, spinal pain (assumed to be due to instability) has been treated by spinal fusion, with or without instrumentation, which causes the vertebrae above and below the disc to grow solidly together and form a single, solid piece of bone. The procedure is carried out with or without discectomy. Other treatments include discectomy alone or disc decompression with or without fusion.

Nuclectomy can be performed by removing some of the nucleus to reduce pressure on the annulus. However, complications include disc space infection, nerve root injury, hematoma formation, and instability of adjacent vertebrae.

These interventions have been problematic in that alleviation of back pain is unpredictable even if surgery appears successful. In attempts to overcome these difficulties, new fixation devices have been introduced to the market, including but not limited to pedicle screws and interbody fusion cages. Although pedicle screws provide a high fusion success rate, there is still no direct correlation between fusion success and patient improvement in function and pain. Studies on fusion have demonstrated success rates of between 50% and 67% for pain improvement, and a significant number of patients have more pain postoperatively. Therefore, different methods of helping patients with degenerative disc problems need to be explored.

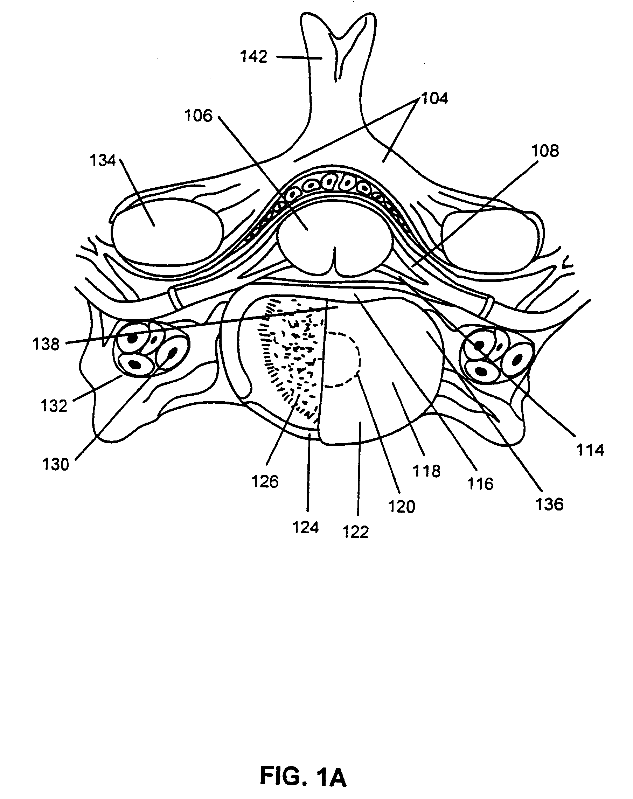

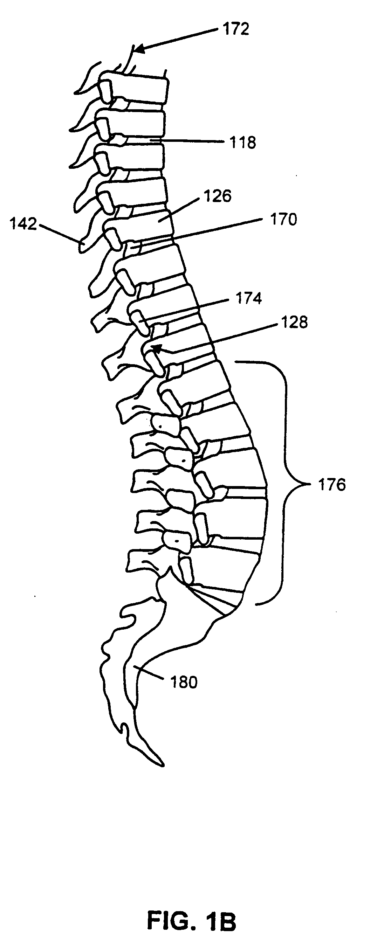

FIGS. 1A and 1B illustrate a cross-sectional anatomical view of a vertebra and associated disc and a lateral view of a portion of a lumbar and thoracic spine, respectively. Structures of a typical cervical vertebra (superior aspect) are shown in FIG. 1A: 104 - lamina; 106 - spinal cord; 108 - dorsal root of spinal nerve; 114 - ventral root of spinal nerve; 116 - posterior longitudinal ligament; 118 - intervertebral disc; 120 - nucleus pulposus; 122 - annulus fibrosus; 124 - anterior longitudinal ligament; 126 - vertebral body; 128 - pedicle; 130 - vertebral artery; 132 - vertebral veins; 134 - superior articular facet; 136 - posterior lateral portion of the annulus; 138 - posterior medial portion of the annulus; and 142 - spinous process. In FIG. 1A, one side of the intervertebral disc 118 is not shown so that the anterior vertebral body 126 can be seen. FIG. 1B is a lateral aspect of the lower portion of a typical spinal column showing the entire lumbar region and part of the thoracic region and displaying the following structures: 118 - intervertebral disc; 126 - vertebral body; 142 - spinous process; 170 - inferior vertebral notch; 172 - spinal nerve; 174 - superior articular process; 176 - lumbar curvature; and 180 - sacrum.

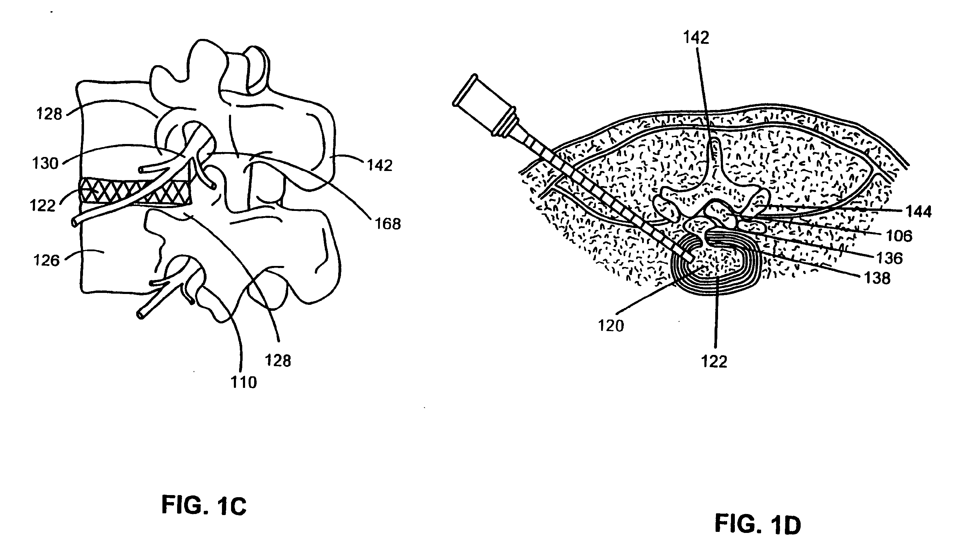

The presence of the spinal cord (nerve sac) and the posterior portion of the vertebral body, including the spinous process, and superior and inferior articular processes, prohibit introduction of a needle or trocar from a directly posterior position. This is important because the posterior disc wall is the site of symptomatic annulus tears and disc protrusions/extrusions that compress or irritate spinal nerves for most degenerative disc syndromes. The inferior articular process, along with the pedicle and the lumbar spinal nerve, form a small "triangular" window (shown in black in FIG. 1C) through which introduction can be achieved from the posterior lateral approach. FIG. 1D looks down on an instrument introduced by the posterior lateral approach. It is well known to those skilled in the art that percutaneous access to the disc is achieved by placing an introducer into the disc from this posterior lateral approach, but the triangular window does not allow much room to maneuver. Once the introducer pierces the tough annulus fibrosus, the introducer is fixed at two points along its length and has very little freedom of movement. Thus, this approach has allowed access only to small central and anterior portions of the nucleus pulposus. Current methods do not permit percutaneous access to the posterior half of the nucleus or to the posterior wall of the disc. Major and potentially dangerous surgery is required to access these areas.

U.S. Patent No. 5,433,739 (the '''739 patent") discloses placement of an RF electrode in an interior region of the disc approximately at the center of the disc. RF power is applied, and heat then putatively spreads out globally throughout the disc. The'739 patent teaches the use of a rigid shaft which includes a sharpened distal end that penetrates through the annulus fibrosus and into the nucleus pulposus. In one embodiment the shaft has to be rigid enough to permit the distal end of the RF electrode to pierce the annulus fibrosus, and the ability to maneuver its distal end within the nucleus pulposus is limited. In another embodiment, a somewhat more flexible shaft is disclosed. However, neither embodiment of the devices of the '739 patent permits access to the posterior, posterior lateral and posterior medial region of the disc, nor do they provide for focal delivery of therapy to a selected local region within the disc or precise temperature control at the annulus. The '739 patent teaches the relief of pain by globally heating the disc. There is no disclosure of treating an annular tear or fissure.

U.S. Patent No. 5,201,729 (the "'729 patent") discloses the use of an optical fiber that is introduced into a nucleus pulposus. In the '729 patent, the distal end of a stiff optical fiber shaft extends in a lateral direction relative to a longitudinal axis of an introducer. This prevents delivery of coherent energy into the nucleus pulposus in the direction of the longitudinal axis of the introducer. Due to the constrained access from the posterior lateral approach, stiff shaft and lateral energy delivery, the device of the '729 patent is unable to gain close proximity to selected portion(s) of the annulus (i.e., posterior, posterior medial and central posterior) requiring treatment or to precisely control the temperature at the annulus. No use in treating an annular fissure is disclosed.

### SUMMARY OF THE INVENTION

Accordingly, it is desirable to diagnose and treat disc abnormalities such as disc degeneration at locations previously not accessible via percutaneous approaches and without major surgical intervention or substantial destruction to the disc. It would be further desirable to treat disc abnormalities via controlled high-energy input available through radio frequency energy. It would be further desirable to provide such RF energy to the nucleus pulposus at the posterior, posterior lateral and the posterior medial regions of the inner wall of the annulus fibrosis, without heating other regions of the nucleus, as would occur with prior art heating elements. It would further be desirable to be able to administer materials to, or remove materials from, a precise, selected location within the disc, particularly to the location of the annular fissure. It would be further desirable to provide thermal energy into collagen in the area of the fissure to strengthen the annulus and possibly fuse collagen to the sides of the fissure, particularly at the posterior, posterior lateral and the posterior medial regions of the inner wall of the annulus fibrosus.

A primary object of the invention is to provide a minimally invasive method and apparatus for diagnosing and treating fissures of discs at selected locations within the disc.

Another object of the invention is to provide a minimally invasive method and apparatus for treating morphological abnormalities of discs at selected locations within the disc via radio frequency electrodes.

Another object of the invention is to provide a device which has a distal end that is inserted into the disc and accesses the posterior, posterior lateral and the posterior medial regions of the inner wall of the annulus fibrosis for application of RF energy at such location.

Another object of the invention is to provide an apparatus which is advanceable and navigable at the inner wall of the annulus fibrosus to provide localized heating at the site of the annular fissure.

Another object of the invention include providing apparatus and methods for diagnosing an abnormality and/or adding or removing a material at a preselected location of a disc via a functional element.

Another object of the invention is to provide a device which has a distal end that is inserted into the disc and accesses the posterior, posterior lateral and the posterior medial regions of the inner wall of the annulus fibrosus in order to repair or shrink an annular fissure at such a location.

Another object of the invention is to provide a non-destructive method and apparatus for treating morphologic abnormalities of discs.

Another object of the invention is to provide a method and apparatus to treat degenerative intervertebral discs by delivering thermal energy to denervate selective nerves embedded in the walls of the disc.

Another objective of the invention is to provide a method and apparatus to treat degenerative intervertebral discs by delivering thermal energy to cauterize granulation tissue that is ingrown in the wall of the disc.

Another object of the invention is to provide a method and apparatus to treat degenerative intervertebral discs by delivering thermal energy to break down selected enzyme systems and neurotransmitters that generate pain within the disc.

Another object of the invention is to provide a method and apparatus to treat degenerative intervertebral discs by shrinking a selected amount of collagen in the annulus fibrosis of the disc and remove a redundancy in the disc roll.

Another object of the invention is to provide a method and apparatus to treat degenerative intervertebral discs by delivering thermal energy to at least a portion of the nucleus pulposus to reduce water content of the nucleus pulposus and shrink the nucleus pulposus without creating a contained herniated disc.

Another object of the invention is to provide a method and apparatus to treat degenerative intervertebral discs by supplying sufficient thermal energy to shrink the nucleus pulposus and tighten the disc.

Another object of the invention is to provide an apparatus to treat degenerative intervertebral discs which is advanceable and navigational adjacent to an inner wall of the annulus fibrosis.

Another object of the invention is to provide a thermal energy delivery device which has a distal end that is inserted into the nucleus pulposus and accesses the posterior, posterior lateral and the posterior central regions of the inner wall of the nucleus fibrosis.

The invention provides an intervertebral disc apparatus that includes an introducer with an introducer lumen and a catheter. The catheter is at least partially positioned in the introducer lumen and includes an intradiscal section and an energy delivery device coupled to the intradiscal section. The intradiscal section is configured to be advanceable through a nucleus pulposus of the intervertebral disc and positionable adjacent to a selected site of an inner wall of an annulus fibrosis. The energy delivery device is configured to deliver sufficient energy to heat at least a portion of the intervertebral disc without substantially removing intervertebral disc material positioned adjacent to the energy delivery device.

The invention also includes providing an externally guidable intervertebral disc apparatus for manipulation of disc tissue present at a preselected location of an intervertebral disc, the disc having a nucleus pulposus, an annulus fibrosis, and an inner wall of the annulus fibrosis, the nucleus pulposus having a first diameter and a disc playing between opposing sections of the inner wall, proximity to the nucleus being provided by an introducer comprising an internal introducer lumen with an opening at a terminus of the introducer, comprising a catheter having a distal end and a proximal end having a longitudinal access, the catheter being adapted to slidably advance through the introducer lumen, the catheter having an intradiscal section at the distal end of the catheter, the intradiscal section being extendable through the opening of the introducer and having sufficient rigidity to be advanceable through the nucleus pulposus of the disc and around the inner wall of the annulus fibrosis under a force applied longitudinally to the proximal end and having insufficient penetration ability to be advanceable through the inner wall of the annulus fibrosis under the force; and a heating element located at the intradiscal section selected from the group consisting of RF heating elements, resistive heating elements, chemical heating elements, and ultrasound heating elements.

The invention also provides methods for manipulating a disc tissue with a fissure or tear in an intervertebral disc, the disc having a nucleus pulposus and an annulus fibrosus, and the annulus having an inner wall of the annulus fibrosus. The method employs an externally guidable intervertebral disc apparatus, or catheter. The procedure is performed with a catheter having a distal end, a proximal end, a longitudinal axis, and an intradiscal section at the catheter's distal end on which there is at least one functional element. The catheter is advanced through the nucleus pulposus and around an inner wall of an annulus fibrosus by applying a force to the proximal end, but the applied force is insufficient for the intradiscal section to puncture the annulus fibrosus. The next step is positioning the functional element at a selected location of the disc by advancing or retracting the catheter and optionally twisting the proximal end of the catheter. Then the functional unit treats the annular fissure.

The invention also includes a method of manipulating disc tissue at a preselected location of an intervertebral disc, the disc having a nucleus pulposus, an annulus fibrosis, and an inner wall of the annulus fibrosis, the nucleus pulposus having a first diameter in a disc playing between opposing sections of the inner wall, comprising providing an introducer with a proximal end and a distal end and having an introducer lumen with a distal opening at a terminus of the introducer, the introducer being located so that the proximal end of the introducer is external to the body and the distal opening of the introducer lumen is internal to the body and (1) internal to the annulus pulposus or (2) adjacent to an opening in the annulus fibrosis communicating with the nucleus pulposus; slidably positioning a catheter having a distal end and a proximal end and having a longitudinal access in the introducer lumen, the catheter having sufficient rigidity to be advanceable through the nucleus pulposus of the disc and around the inner wall of the annulus fibrosis under a force applied longitudinally to the proximal end and having insufficient penetration ability to be advanceable through the inner wall of the annulus fibrosis under the force; positioning a heating element in the catheter at the preselected location of the disc by advancing or retracting the catheter in the introducer lumen and optionally twisting the proximal end of the catheter; and heating the preselected location of the disc via the heating element, wherein the heating element is selected from the group consisting of RF heating elements, resistive heating elements, chemical heating elements, and acoustical heating elements.

The invention also includes a method of treating an intervertebral fissure comprises the steps of placing an energy source adjacent to the fissure and providing sufficient energy to the fissure to raise the temperature to at least about 45-70°C and for a sufficient time to cause the collagen to weld.

The invention also includes a method of treating an intervertebral fissure comprises placing a catheter with a lumen adjacent to the fissure and injecting sealant into the fissure via the catheter lumen to seal the fissure.

The invention also includes providing an externally guidable intervertebral disc apparatus for diagnosis or manipulation of disc tissue present at a selected location of an intervertebral disc, the disc having a nucleus pulposus, an annulus fibrosus, and an inner wall of the annulus fibrosus, and the nucleus pulposus having a diameter in a disc plane between opposing sections of the inner wall. The apparatus comprises a catheter having a distal end, a proximal end, and a longitudinal axis, and an intradiscal section at the catheter's distal end, which is extendible into the disc, has sufficient rigidity to be advanceable through the nucleus pulposus and around the inner wall of the annulus fibrosus under a force applied longitudinally to the proximal end, has sufficient flexibility in a direction of the disc plane to be compliant with the inner wall, and has insufficient penetration ability to be advanceable out through the annulus fibrosus under the force; and a functional element located at the intradiscal section for adding sufficient thermal energy at or near the fissure.

### BRIEF DESCRIPTION OF THE FIGURES

The invention will be better understood by reference to the following figures that form part of the specification, wherein:
FIG. 1 A is a superior cross-sectional anatomical view of a cervical disc and vertebra.
FIG. 1B is a lateral anatomical view of a portion of a lumbar spine.
FIG. 1C is a posterior-lateral anatomical view of two lumbar vertebrae and an illustration of the triangular working zone, representing an embodiment of the present invention.
FIG. 1D is a superior cross-sectional view of the required posterior lateral approach, representing an embodiment of the present invention.
FIG. 2 is a cross-sectional view of an intervertebral disc illustrating a disc plane of the intervertebral disc and an inferior/superior plane, representing an embodiment of the present invention.
FIG. 3A is a plan view of an introducer and an instrument of the invention in which solid lines illustrate the position of the instrument in the absence of bending forces and dotted lines indicate the position the distal portion of the instrument would assume under bending forces applied to the intradiscal section of the instrument, representing an embodiment of the present invention.
FIG. 3B is an end view of the handle of the embodiment shown in FIG. 3A
FIG. 4 is a cross-sectional view of an intervertebral disc with a portion of the intervertebral apparatus of the present invention inserted in the intervertebral disc, representing an embodiment of the present invention.
FIG. 5A is a cross-sectional view of the intervertebral segment of the embodiment of the invention shown in FIG. 3A taken along the line 5(a)-5(a) of FIG. 3A.
FIG. 5B is a cross-sectional view of the intervertebral segment of an embodiment of the present invention having a combined wall/guiding mandrel, representing an embodiment of the present invention.
FIG. 6 is a perspective view of an embodiment of an apparatus of the present invention with a resistive heating coil positioned around an exterior of an intradiscal section of a catheter, representing an embodiment of the present invention.
FIG. 7 is a partial cross-sectional view of an embodiment of an apparatus of the invention illustrating a sensor positioned in an interior of the intradiscal section of the catheter, representing an embodiment of the present invention.
FIG. 8 is a partial cross-sectional view of an embodiment of an apparatus of the invention further including a sheath positioned around the resistive heating coil, representing an embodiment of the present invention.
FIG. 9 is a partial cross-sectional view of an embodiment of the apparatus of FIG. 6 with multiple resistive heating coils, representing an embodiment of the present invention.
FIG. 10 is a plan view of an embodiment of the intradiscal section of a catheter of the invention with a helical structure, representing an embodiment of the present invention.
FIG. 11 is a block diagram of an open or closed loop feedback system that couples one or more sensors to an energy source, representing an embodiment of the present invention.
FIG. 12 is a block diagram of an embodiment illustrating an analog amplifier, analog multiplexer and microprocessor used with the feedback control system of FIG. 11.
FIG. 13 shows a catheter and cannula, representing an embodiment of the present invention.
FIGS. 14A-D show a spinal disc pierced by a cannula through which a catheter is introduced, representing an embodiment of the present invention.
FIGS. 15A-E are detailed views of a cannula, representing an embodiment of the present invention.
FIGS. 16A-B show a stylette-obturator, representing an embodiment of the present invention.
FIG. 17 shows the tip of a cannula with a stylette-obturator, representing an embodiment of the present invention.
FIG. 18 shows a catheter, representing an embodiment of the present invention.
FIGS. 19A-C show various cross-sectional views of the cannula shown in FIG. 18.
FIG. 20 is a cross-sectional view of a self-aligning cannula, representing an embodiment of the present invention.
FIG. 21 is a cross-sectional view of the heater portion of a catheter, representing an embodiment of the present invention.
FIG. 22 shows a cross-sectional view of an alternate embodiment of the heater portion of a catheter, representing an embodiment of the present invention.
FIGS. 23-25 show three alternate embodiments for providing navigational capability to the cannula, representing an embodiment of the present invention.
FIG. 26 is a cross-sectional view of a cannula with a deflection detector, representing an embodiment of the present invention.
FIG. 27 is a cross-sectional view of the handle portion of a cannula showing a heat control and buckle detection circuitry, representing an embodiment of the present invention.
FIGS. 28A-B show respectively a side and cross-sectional view of a catheter for introducing and/or removing materials from a surgical site, representing an embodiment of the present invention.
FIGS. 29A-B are cross-sectional views of alternate embodiments of a catheter for heat and material delivery and/or removal, representing an embodiment of the present invention.
FIG. 30 is an electrical diagram of an analog circuit for providing and controlling temperature delivery, representing an embodiment of the present invention.
FIG. 31 is a signal diagram showing voltage wave forms at specific nodes within the circuit shown in FIG. 30.
FIG. 32 is an electrical diagram showing an alternate embodiment to the analog circuit shown in FIG 30.
FIG. 33 is a signal diagram at specific nodes within the circuit shown in FIG. 32.
FIG. 34 is an electrical diagram showing a digital circuit for temperature delivery and control, representing an embodiment of the present invention.
FIG. 35 is a signal diagram at selected nodes within the circuit shown in FIG. 34.
The invention now being generally described, the same will be better understood by reference to the following detailed description of specific embodiments and general features of the invention.

### DETAILED DESCRIPTION

The present invention provides a method and apparatus for treating intervertebral disc disorders by the application of controlled heating to a localized region of an intervertebral disc. Such disorders include but are not limited to (i) degenerative discs which have tears or fissures in the annulus fibrosis, particularly fissures of the annulus fibrosis, which may or may not be accompanied with contained or escaped extrusions, (ii) contained disc herniations with focal protrusions, and (iii) bulging discs. Such disorders include but are not limited to (i) degenerative discs which have tears or fissures in the annulus fibrosis, (ii) contained disc herniations with focal protrusions, and (iii) bulging discs.

Degenerative discs with tears or fissures are treated non-destructively without the removal of disc tissue other than limited ablation to the nucleus pulposus which changes some of the water content of the nucleus pulposus. Nothing is added to supplement the mechanics of the disc. Electromagnetic energy is delivered to a selected section of the disc in an amount which does not create a destructive lesion to the disc, other than at most a change in the water content of the nucleus pulposus. In one embodiment, there is no removal and/or vaporization of disc material positioned adjacent to an energy delivery device positioned in a nucleus pulposus. Sufficient electromechanical energy is delivered to the disc to change its biochemical, neurophysiologic and/or biomechanical properties. Neurophysiologic modifications include denervation of nociceptores in a tear or fissure in the annulus fibrosis.

Degenerative intervertebral discs with fissures are treated by denervating selected nerves that are embedded in the interior wall of the annulus fibrosis as well as nerves outside of the interior wall including those on the surface of the wall. Electromagnetic energy is used to cauterize granulation tissue which are pain sensitive areas and formed in the annulus fibrosis wall. Electromagnetic energy is also used to break down selected enzyme systems and neurotransmitters that generate pain within the disc. Generally, these enzymes and neurotransmitters only work within a small bandwidth of both pH and temperature.

Electromagnetic energy is applied to shrink collagen in the annulus fibrosis and/or nucleus pulposus. This reduces the redundancy in the disc roll that is created in a degenerative disc. Delivery of electromagnetic energy to the nucleus pulposis removes some water and permits the nucleus pulposis to withdraw. This reduces a "pushing out" effect that created a contained herniation. Combinations of shrinking the disc, shrinking of the nucleus pulposis by reducing water content, as well as tightening up the annulus fibrosis wall creates a rejuvenation of the disc. Reducing the pressure in the disc and tightening the annulus fibrosis produces a favorable biomechanical effect. Application of electromagnetic energy locally increases the stiffness of the disc.

The annulus fibrosis is comprised primarily of fibrosis-like material and the nucleus pulposis is comprised primarily of an amorphous colloidal gel. The distinction between the annulus fibrosis and the nucleus pulposis becomes more difficult to distinguish when a patient is 30 years old or greater. There is often a transition zone between the annulus fibrosis and the nucleus pulposis made of fibrosis-like material and amorphous colloidal gel. For purposes of this disclosure, the inner wall of the annulus fibrosis includes the young wall comprised primarily of fibrosis-like material as well as the transition zone which includes both fibrous-like material and amorphous colloidal gels (hereinafter collectively referred to as "inner wall of the annulus fibrosis").

In general, an apparatus of the invention is in the form of an externally guidable intervertebral disc apparatus for accessing and manipulating disc tissue present at a selected location of an intervertebral disc having a nucleus pulposus and an annulus fibrosus, the annulus having an inner wall. Use of a temperature-controlled energy delivery element, combined with the navigational control of the inventive catheter, provides preferential, localized heating to treat the fissure. For ease of reference to various manipulations and distances described below, the nucleus pulposus can be considered as having a given diameter in a disc plane between opposing sections of the inner wall. This nucleus pulposus diameter measurement allows instrument sizes (and parts of instruments) designed for one size disc to be readily converted to sizes suitable for an instrument designed for a different size of disc.

The operational portion of the apparatus of the invention is brought to a location in or near the disc's fissure using techniques and apparatuses typical of percutaneous interventions. For convenience and to indicate that the apparatus of the invention can be used with any insertional apparatus that provides proximity to the disc, including many such insertional apparatuses known in the art, the term "introducer" is used to describe this aid to the method. An introducer has an internal introducer lumen with a distal opening at a terminus of the introducer to allow insertion (and manipulation) of the operational parts of the apparatus into (and in) the interior of a disc.

The operational part of the apparatus comprises an elongated element referred to as a catheter, various parts of which are located by reference to a distal end and a proximal end at opposite ends of its longitudinal axis. The proximal end is the end closest to the external environment surrounding the body being operated upon (which may still be inside the body in some embodiments if the catheter is attached to a handle insertable into the introducer). The distal end of the catheter is intended to be located inside the disc under conditions of use. The catheter is not necessarily a traditional medical catheter (i.e., an elongate hollow tube for admission or removal of fluids from an internal body cavity) but is a defined term for the purposes of this specification. "Catheter" has been selected as the operant word to describe this part of the apparatus, as the inventive apparatus is a long, flexible tube which transmits energy and/or material from a location external to the body to a location internal to the disc being accessed upon, such as a collagen solution and heat to the annular fissure. Alternatively, material can be transported in the other direction to remove material from the disc, such as removing material by aspiration to decrease pressure which is keeping the fissure open and aggravating the symptoms due to the fissure.

The catheter is adapted to slidably advance through the introducer lumen, the catheter having an intradiscal section at the distal end of the catheter, the intradiscal section being extendible through the distal opening at the terminus of the introducer into the disc. Although the length of the intradiscal portion can vary with the intended function as explained in detail below, a typical distance of extension is at least one-half the diameter of the nucleus pulposus, preferably in the range of one-half to one and one-half times the circumference of the nucleus.

In order that the functional elements of the catheter (e.g., an electromagnetic probe, such as, an RF electrode or a resistance heater) can be readily guided to the desired location within a disc, the intradiscal portion of the catheter is manufactured with sufficient rigidity to avoid collapsing upon itself while being advanced through the nucleus pulposus and navigated around the inner wall of the annulus fibrosus. The intradiscal portion, however, has insufficient rigidity to puncture the annulus fibrosus under the same force used to advance the catheter through the nucleus pulposus and around the inner wall of the annulus fibrosus. Absolute penetration ability will vary with sharpness and stiffness of the tip of the catheter, but in all cases a catheter of the present invention will advance more readily through the nucleus pulposus than through the annulus fibrosus.

In preferred embodiments, the intradiscal section of the catheter further has differential bending ability in two orthogonal directions at right angles to the longitudinal axis. This causes the catheter to bend along a desired plane (instead of at random). Also when a torsional (twisting) force is applied to the proximal end of the catheter to re-orient the distal end of the catheter, controlled advancement of the catheter in the desired plane is possible.

A further component of the catheter is a functional element located in the intradiscal section for diagnosis or for adding energy and adding and/or removing material at the selected location of the disc where the annular tear is to be treated. The apparatus allows the functional element to be controllably guided by manipulation of the proximal end of the catheter into a selected location for localized treatment of the annular fissure.

The method of the invention, which involves manipulating disc tissue at the annular fissure, is easily carried out with an apparatus of the invention. An introducer is provided that is located in a patient's body so that its proximal end is external to the body and the distal opening of its lumen is internal to the body and (1) internal to the annulus fibrosus or (2) adjacent to an annular opening leading to the nucleus pulposus, such as an annular tear or trocar puncture that communicates with the nucleus pulposus. The catheter is then slid into position in and through the introducer lumen so that the functional element in the catheter is positioned at the selected location of the disc by advancing or retracting the catheter in the introducer lumen and optionally twisting the proximal end of the catheter to precisely navigate the catheter. By careful selection of the rigidity of the catheter and by making it sufficiently blunt to not penetrate the annulus fibrosus, and by careful selection of the flexibility in one plane versus the orthogonal plane, the distal portion of the catheter will curve along the inner wall of the annulus fibrosus as it is navigated and is selectively guided to an annular tear at selected location(s) in the disc. Energy is applied and/or material is added or removed at the selected location of the disc via the functional element.

Each of the elements of the apparatus and method will now be described in more detail. However, a brief description of disc anatomy is provided first, as sizes and orientation of structural elements of the apparatus and operations of the method can be better understood in some cases by reference to disc anatomy.

The annulus fibrosus is comprised primarily of tough fibrous material, while the nucleus pulposus is comprised primarily of an amorphous colloidal gel. There is a transition zone between the annulus fibrosus and the nucleus pulposus made of both fibrous-like material and amorphous colloidal gel. The border between the annulus fibrosus and the nucleus pulposus becomes more difficult to distinguish as a patient ages, due to degenerative changes. This process may begin as early as 30 years of age. For purposes of this specification, the inner wall of the annulus fibrosus can include the young wall comprised primarily of fibrous material as well as the transition zone which includes both fibrous material and amorphous colloidal gels (hereafter collectively referred to as the "inner wall of the annulus fibrosus"). Functionally, that location at which there is an increase in resistance to catheter penetration and which is sufficient to cause bending of the distal portion of the catheter into a radius less than that of the internal wall of the annulus fibrosus is considered to be the "inner wall of the annulus fibrosus."

As with any medical instrument and method, not all patients can be treated, especially when their disease or injury is too severe. There is a medical gradation of degenerative disc disease (stages 1-5). See, for example, Adams et al., "The Stages of Disc Degeneration as Revealed by Discograms," J. Bone and Joint Surgery, 68, 36-41 (1986). As these grades are commonly understood, the methods of instrument navigation described herein would probably not be able to distinguish between the nucleus and the annulus in degenerative disease of grade 5. In any case, most treatment is expected to be performed in discs in stages 3 and 4, as stages 1 and 2 are asymptomatic in most patients, and stage 5 may require disc removal and fusion.

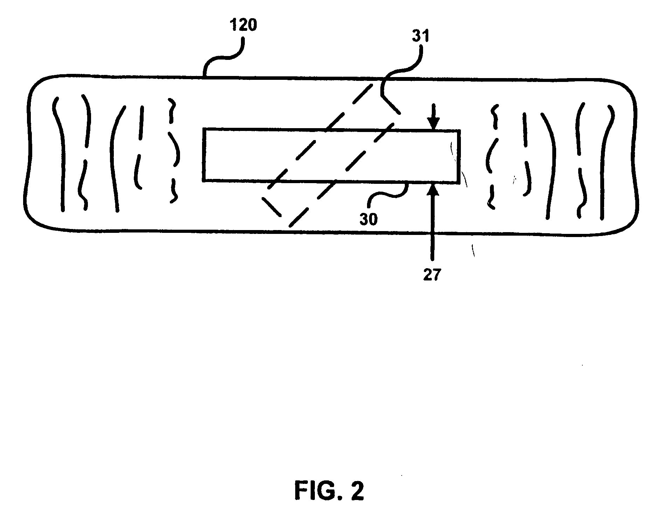

Some of the following discussion refers to motion of the catheter inside the disc by use of the terms "disc plane," "oblique plane" and "cephalo-caudal plane." These specific terms refer to orientations of the catheter within the intervertebral disc. Referring now to FIG. 2 (which shows a vertical cross-section of a disc), a disc plane 30 of the intervertebral disc is generally a plane of some thickness 27 within the nucleus pulposus 120 orthogonal to the axis formed by the spinal column (i.e., such a disc plane is substantially horizontal in a standing human, corresponding to the "flat" surface of a vertebra). A oblique plane 31 extends along any tilted orientation relative to axial plane 30; however, when the plane is tilted 90°, such a plane would be substantially vertical in a standing human and is referred to as a cephalo-caudal plane. Reference is made to such planes to describe catheter movements with respect to the disc plane. In various embodiments, disc plane 30 has a thickness no greater than the thickness of the intervertebral disc, preferably a thickness no greater than 75% of a thickness of the intervertebral disc, and more preferably a thickness no greater than 50% of a thickness of the intervertebral disc. Movement of the intradiscal portion 16 of catheter 14 is confined within a disc plane by the physical and mechanical properties of the intradiscal portion 16 during advancement of the catheter when the bending plane of the catheter is aligned with the disc plane, until some additional force is applied to the catheter by the physician. A twisting force (which can be applied mechanically, electrically, or by any other means) acting on the proximal end of the catheter changes the forces acting on the distal end of the catheter so that the plane of the catheter bend can be angled relative to the disc plane as the catheter is advanced. Thus, the physician can cause the distal end of the catheter to move up or down, depending on the direction of the twist.

Turning now to the introducer, a detailed description of an entire apparatus should not be necessary for those skilled in the art of percutaneous procedures and the design of instruments intended for such use. The method of the invention can also be carried out with endoscopic instruments, and an endoscopic apparatus having structural parts that meet the descriptions set forth in this specification would also be an apparatus of the invention.

In general, a device of the invention can be prepared in a number of different forms and can consist (for example) of a single instrument with multiple internal parts or a series of instruments that can be replaceably and sequentially inserted into a hollow fixed instrument (such as a needle) that guides the operational instruments to a selected location in or adjacent to an annular fissure. Because prior patents do not fully agree on how to describe parts of percutaneous instruments, terminology with the widest common usage will be used.

Because the present invention is directed to navigation of an instrument through the nucleus pulposus, particularly to the moveable distal portion of the probe, and not to the instrument or instruments used to access the nucleus pulposus, the general term "introducer" is used to refer to the instrument that provides access to the nucleus. The form of the introducer is not a material part of the present invention and can vary.

The introducer, in its simplest form, can consist of a hollow needle-like device (optionally fitted with an internal removable obturator or trocar to prevent clogging during initial insertion, variously known as a stylet, obturator, or trocar, depending on its shape and other functions) or a combination of a simple exterior cannula that fits around a trocar. The result is essentially the same: placement of a hollow tube (the needle or exterior cannula after removal of the obturator or trocar, respectively) through skin and tissue to provide access into the annulus fibrosus. The hollow introducer acts as a guide for introducing instrumentation.

More complex variations exist in percutaneous instruments designed for other parts of the body and can be applied to design of instruments intended for disc operations. For example, in many cases the exterior cannula of a trocar is replaced by an obturator, by which is meant any instrument that acts to seal off the interior of the body from the external environment after withdrawal of the trocar. Examples of such obturators are well known in the art. A particularly preferred introducer is a 17- or 18-gauge, thin-wall needle with a matched obturator, which after insertion is replaced with a catheter of the present invention.

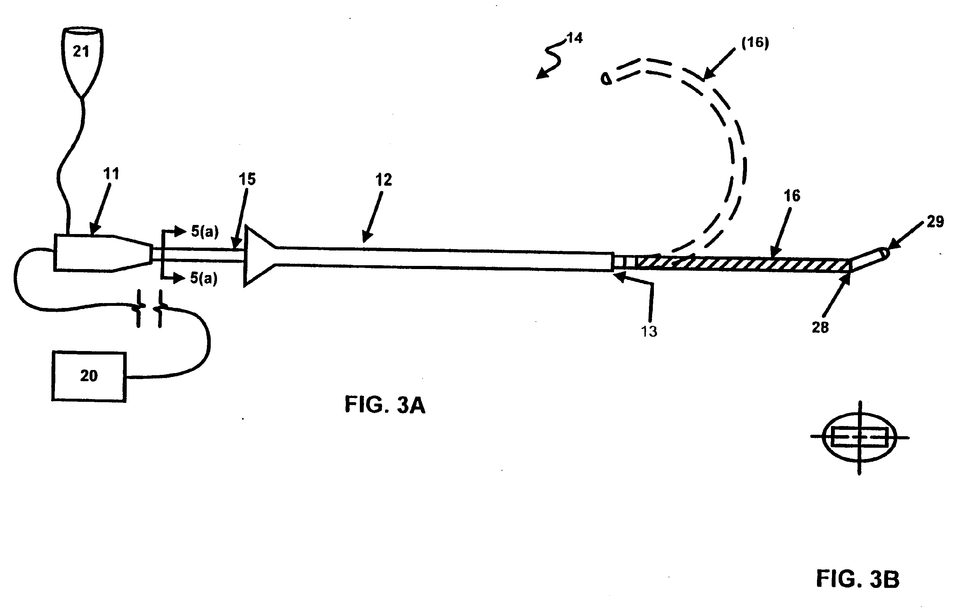

Referring now to the figures, FIGS. 3A and 3B illustrate one embodiment of a catheter 14 of the invention as it would appear inserted into an introducer 12. The apparatus shown is not to scale, as an exemplary apparatus (as will be clear from the device dimensions below) would be relatively longer and thinner; the proportions used in FIG. 3A were selected for easier viewing by the reader. The distal portion of an intervertebral apparatus operates inside an introducer 12 having an internal introducer lumen 13. A flexible, movable catheter 14 is at least partially positionable in the introducer lumen 13. Catheter 14 includes a distal end section 16 referred to as the intradiscal section, which is designed to be the portion of the catheter that will be pushed out of the introducer lumen and into the nucleus pulposus, where movement of the catheter will be controlled to bring operational portions of the catheter into the selected location(s) with regard to the annular tear. In FIG. 3A, dashed lines are used to illustrate bending of the intradiscal portion of the catheter as it might appear under use, as discussed in detail later in the specification. FIG. 3B shows an end view of handle 11 at the proximal end of the catheter, with the handle 11 having an oval shape to indicate the plane of bending, also discussed in detail later in the specification. Other sections and properties of catheter 14 are described later.

For one embodiment suitable for intervertebral discs, the outer diameter of catheter 14 is in the range of 0.2 to 5 mm, the total length of catheter 14 (including the portion inside the introducer) is in the range of 10 to 60 cm, and the length of introducer 12 is in the range of 5 to 50 cm. For one preferred embodiment, the catheter has a diameter of 1 mm, an overall length of 30 cm, and an introduced length of 15 cm (for the intradiscal section). With an instrument of this size, a physician can insert the catheter for a distance sufficient to reach selected location(s) in the nucleus of a human intervertebral disc.

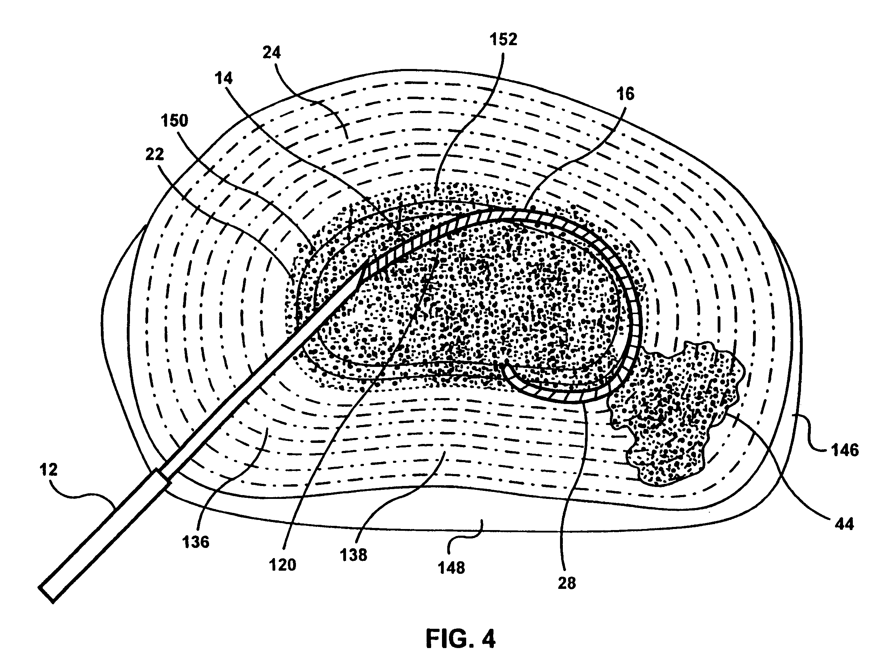

FIG. 4 illustrates the anatomy of an intervertebral disc and shows an apparatus of the invention inserted into a disc. Structures of the disc are identified and described by these anatomical designations: the posterior lateral inner annulus 136, posterior medial inner annulus 138, annulus fibrosus 122/nucleus pulposus 120 interface, the annulus/dural interface 146, annulus/posterior longitudinal ligament interface 148, anterior lateral inner annulus 150, and the anterior medial inner annulus 152.

Referring again to FIG. 4, the mechanical characteristics of intradiscal section 16 of catheter 14 are selected to have (1) sufficient column strength along the longitudinal axis of the catheter to avoid collapse of the catheter and (2) different flexural strengths along the two axes orthogonal to the longitudinal axis to allow controlled bending of the catheter. These parameters make the catheter conformable and guidable along inner wall 22 of an annulus fibrosus 122 to reach selected location(s), such as the posterior medial annulus 138.

Specific mechanical characteristics of particular designs will be described later in the examples that follow. Generally, however, the necessary design features can be selected (in an interrelated fashion) by first providing the intradiscal section of the catheter with sufficient column strength to be advanceable through normal human nucleus pulposus and around the inner wall of the annulus fibrosus without collapse. Here "collapse" refers to bending sufficient to inhibit further advancement at the tip. Advancement of the tip is restricted by 1) sliding through the normal gelatinous nucleus pulposus, 2) contacting denser clumps of nucleus pulposus, and 3) curving and advancing along the inner wall of the annulus. Column strength can be increased in many ways known in the art, including but not limited to selecting materials (e.g., metal alloy or plastic) with a high resistance to bending from which to form the catheter, forming the structure of the catheter with elements that add stiffening (such as bracing), and increasing the thickness of the structural materials. Column strength can be decreased to favor bending by selecting the opposite characteristics (e.g., soft alloys, hinging, and thin structural elements).

When the catheter collapses, the physician feels an abrupt decrease in resistance. At that time, the catheter forms one or more loops or kinks between the tip of the introducer and the distal tip of the catheter.

In some embodiments, particularly those that are preferred for annular tears at the posterior of the annulus, the tip 28 of intradiscal section 16 is biased or otherwise manufactured so that it forms a pre-bent segment prior to contact with the annulus fibrosus as shown in FIG. 3A. The bent tip will cause the intradiscal section to tend to continue to bend the catheter in the same direction as the catheter is advanced. This enhanced curving of a pre-bent catheter is preferred for a catheter that is designed to reach a posterior region of the nucleus pulposus; however, such a catheter must have sufficient column strength to prevent the catheter from collapsing back on itself.

The intradiscal section not only must allow bending around the relatively stronger annulus fibrosus in one direction, but also resist bending in the orthogonal direction to the plane in which bending is designed to occur. By twisting the proximal end of a catheter and thus controlling the orientation of the plane of bending while concurrently controlling the advancement of the catheter through the nucleus, a physician can navigate the catheter and its instrumentation within the disc.

The bending stiffness of the intradiscal section as measured in Taber stiffness units (using a length of the inventive catheter as the test strip rather than the standard dimension, homogeneous-material test strip) should be in the range of 2-400 units (in a 0-10,000 unit range) in the desired bending plane, preferably 3-150 units. In preferred embodiments, stiffness is in the range of 4-30 units in the desired bending plane. In all cases, the bending stiffness preferably is 2-20 times higher for bending in the orthogonal direction.

The column or compressive strength of the intradiscal section (force required to buckle a segment whose length is 25 or more times its diameter) is in the range of 0.05 to 4 kg, preferably 0.05 to 2 kg. In the most preferred embodiments, it is in the range of 0.1 to 1 kg. In the proximal shaft section (i.e., the part of the catheter proximal to the intradiscal section), this strength is in the range of 0.1 to 25 kg, preferably 0.2 to 7 kg. In the most preferred embodiments, it is in the range of 0.7 to 4 kg.

Returning now to FIG. 4, intradiscal section 16 is guidable and can reach the posterior, the posterior lateral, and the posterior medial regions of the posterior wall of the annulus fibrosus, as well as other selected section(s) on or adjacent to inner wall 22. In order to move the functional section of the catheter into a desired nucleus location, intradiscal section 16 is first positioned in the nucleus pulposus 120 by means of the introducer.

In most uses, introducer 12 pierces annulus fibrosus 122 and is advanced through the wall of the annulus fibrosus into the nucleus pulposus. In such embodiments, introducer 12 is then extended a desired distance into nucleus pulposus 120. Catheter 14 is advanced through a distal end of introducer 12 into nucleus pulposus 120. Advancement of the catheter 14, combined with increased resistance to advancement at the annulus fibrosus, causes the tip of the intradiscal section to bend relative to the longitudinal axis of introducer 12 when the intradiscal section contacts the inner wall of the annulus fibrosus 122. Catheter 14 is navigated along inner wall 22 of annulus fibrosus 122 to selected site(s) of inner wall 22 or within nucleus pulposus 120. For example, intradiscal section 16 can be positioned in or adjacent to a fissure or tear 44 of annulus fibrosus 122.

The distal portion 28 of intradiscal section 16 is designed to be incapable of piercing the annulus fibrosus 122. The inability of distal portion 28 to pierce the annulus can be the result of either shape of the tip 29 or flexibility of distal portion 28, or both. The tip 29 is considered sufficiently blunt when it does not penetrate the annulus fibrosus but is deflected back into the nucleus pulposus or to the side around the inner wall of the annulus when the tip 29 is advanced. The tip can be made with a freely rotating ball. This embodiment provides not only a blunt surface but also a rolling contact to facilitate navigation.

Many percutaneous and endoscopic instruments designed for other purposes can be adapted for use in this invention. This permits other functions at the desired location after the catheter is advanced to that position. For example, cutting edges and sharp points can be present in the distal portion 28 if they can be temporarily masked by a covering element. Internally located cutting elements are present in many percutaneous and endoscopic instruments designed for other purposes and can be adapted for use in the instant invention. This would permit cutting at the desired location after the catheter is advanced to that position. However, such devices must be sufficiently flexible and thin to meet the design characteristics described in this specification.

In another embodiment an introducer 12 pierces the skin and reaches an exterior of annulus fibrosus 122. A rigid and sharp trocar is then advanced through introducer 12, to pierce annulus fibrosus 122 and enter the disc. The trocar is then removed, and catheter 14 is advanced through a distal end of introducer 12, following the path created by the trocar in annulus fibrosus 122 beyond the end of the introducer. In such cases, the introducer is outside the annulus fibrosus 122 and only the catheter with its guidable distal portion 16 is present inside the disc. The physician can manipulate the proximal portion 15 of the catheter to move the distal portion of the catheter to a selected location for treating a fissure of the annulus fibrosus 122.

Catheter 14 is not always pre-bent as shown in FIG. 3A, but optionally can include a biased distal portion 28 if desired. "Pre-bent" or "biased" means that a portion of the catheter (or other structural element under discussion) is made of a spring-like material that is bent in the absence of external stress but which, under selected stress conditions (for example, while the catheter is inside the introducer), is linear. Such a biased distal portion can be manufactured from either spring metal or superelastic memory material (such as Tinel® nickel-titanium alloy, Raychem Corp., Menlo Park CA). The introducer (at least in the case of a spring-like material for forming the catheter) is sufficiently strong to resist the bending action of the bent tip and maintain the biased distal portion in alignment as it passes through the introducer. Compared to unbiased catheters, a catheter with a biased distal portion 28 encourages advancement of intradiscal section 16 substantially in the direction of the bend relative to other lateral directions as shown by the bent location of intradiscal section 16 indicated by dashed lines in FIG. 3A Biasing the catheter tip also further decreases likelihood that the tip 29 will be forced through the annulus fibrosus under the pressure used to advance the catheter.

In addition to biasing a catheter tip prior to insertion into an introducer, a catheter tip can be provided that is deflected by mechanical means, such as a wire attached to one side of the tip that deflects the tip in the desired direction upon application of force to the proximal end of the deflection wire. Any device in which bending of the tip of a catheter of the invention is controlled by the physician is "actively steerable." In addition to a tip that is actively steerable by action of a wire, other methods of providing a bending force at the tip can be used, such as hydraulic pressure and electromagnetic force (such as heating a shaped memory alloy to cause it to contract). Any of a number of techniques can be used to provide selective bending of the catheter in one lateral direction.

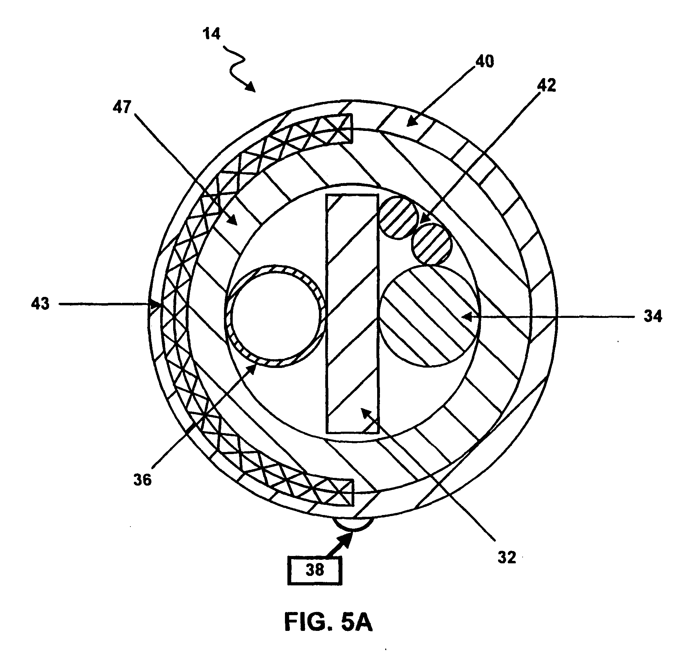

Referring now to FIG. 5A, a guiding mandrel 32 can be included both to add rigidity to the catheter and to inhibit movement of catheter 14 in the inferior and superior directions while positioned and aligned in the disc plane of a nucleus pulposus 120. This aids the functions of preventing undesired contact with a vertebra and facilitating navigation. The mandrel can be flattened to encourage bending in a plane (the "plane of the bend") orthogonal to the "flat" side of the mandrel. "Flat" here is a relative term, as the mandrel can have a D-shaped cross-section, or even an oval or other cross-sectional shape without a planar face on any part of the structure. Regardless of the exact configuration, bending will preferentially occur in the plane formed by the principal longitudinal axis of the mandrel and a line connecting the opposite sides of the shortest cross-sectional dimension of the mandrel (the "thin" dimension). To provide sufficient resistance to the catheter bending *out* of the desired plane while encouraging bending *in* the desired plane, the minimum ratio is 1.25:1 ("thickest" to "thinnest" cross-sectional dimensions along at least a portion of the intradiscal section). The maximum ratio is 20: 1, with the preferred ratio being between 1.5:1 and 16:3, more preferably between 2:1 and 3.5: 1. These ratios are for a solid mandrel and apply to any material, as deflection under stress for uniform solids is inversely proportional to the thickness of the solid in the direction (dimension) in which bending is taking place. For other types of mandrels (e.g., hollow or non-uniform materials), selection of dimensions and/or materials that provide the same relative bending motions under stress are preferred.

A catheter of the present invention is designed with sufficient torsional strength (resistance to twisting) to prevent undesired directional movement of the catheter. Mandrels formed from materials having tensile strengths in the range set forth in the examples of this specification provide a portion of the desired torsional strength. Other materials can be substituted so long as they provide the operational functions described in the examples and desired operating parameters.

While the mandrel can provide a significant portion of the column strength, selective flexibility, and torsional strength of a catheter, other structural elements of the catheter also contribute to these characteristics. Accordingly, it must be kept in mind that it is the characteristics of the overall catheter that determine suitability of a particular catheter for use in the methods of the invention. For example, a mandrel that does not have sufficient torsional strength when acting alone can be combined with another element, such as anti-twisting outer sheath 40 or inserting/advancing a second mandrel, to provide a catheter of the invention. Similarly, components inside the catheter, such as a heating element or potting compound, can be used to strengthen the catheter or provide directional flexibility at the locations of these elements along the catheter.

It is not necessary that the guiding mandrel 32 be flattened along its entire length. Different mandrels can be designed for different sized discs, both because of variations in disc sizes from individual to individual and because of variations in size from disc to disc in one patient. The bendable portion of the mandrel is preferably sufficient to allow intradiscal portion 16 of the catheter to navigate at least partially around the circumference of the inner wall of the annulus fibrosus (so that the operational functions of the catheter can be carried out at desired location(s) along the inner wall of the annulus fibrosus). Shorter bendable sections are acceptable for specialized instruments. In most cases, a flattened distal portion of the mandrel of at least 10 mm, preferably 25 mm, is satisfactory. The flattened portion can extend as much as the entire length of the mandrel, with some embodiments being flattened for less than 15 cm, in other cases for less than 10 cm, of the distal end of the guide mandrel.

In preferred embodiments, the guide mandrel or other differential bending control element is maintained in a readily determinable orientation by a control element located at the proximal end of the catheter. The orientation of the direction of bending and its amount can be readily observed and controlled by the physician. One possible control element is simply a portion of the mandrel that extends out of the proximal end of the introducer and can be grasped by the physician, with a shape being provided that enables the physician to determine the orientation of the distal portion by orientation of the portion in the hand. For example, a flattened shape can be provided that mimics the shape at the distal end (optionally made larger to allow better control in the gloved hand of the physician, as in the handle 11 of FIG. 3B). More complex proximal control elements capable of grasping the proximal end of the mandrel or other bending control element can be used if desired, including but not limited to electronic, mechanical, and hydraulic controls for actuation by the physician.

The guide mandrel can also provide the function of differential flexibility by varying the thickness in one or more dimensions (for example, the "thin" dimension, the "thick" dimension, or both) along the length of the mandrel. A guide mandrel that tapers (becomes gradually thinner) toward the distal tip of the mandrel will be more flexible and easier to bend at the tip than it is at other locations along the mandrel. A guide mandrel that has a thicker or more rounded tip than more proximal portions of the mandrel will resist bending at the tip but aid bending at more proximal locations. Thickening (or thinning) can also occur in other locations along the mandrel. Control of the direction of bending can be accomplished by making the mandrel more round, i.e., closer to having 1:1 diameter ratios; flatter in different sections of the mandrel; or by varying the absolute dimensions (increasing or decreasing the diameter). Such control over flexibility allows instruments to be designed that minimize bending in some desired locations (such as the location of connector of an electrical element to avoid disruption of the connection) while encouraging bending in other locations (e.g., between sensitive functional elements). In this manner, a catheter that is uniformly flexible along its entire length, is variably flexibility along its entire length, or has alternating more flexible and less flexible segment(s), is readily obtained simply by manufacturing the guide mandrel with appropriate thickness at different distances and in different orientations along the length of the mandrel. Such a catheter will have two or more different radii of curvature in different segments of the catheter under the same bending force.

In some preferred embodiments, the most distal 3 to 40 mm of a guide mandrel is thinner relative to central portions of the intradiscal section to provide greater flexibility, with the proximal 10 to 40 mm of the intradiscal section being thicker and less flexible to add column strength and facilitate navigation.

The actual dimensions of the guide mandrel will vary with the stiffness and tensile strength of the material used to form the mandrel. In most cases the mandrel will be formed from a metal (elemental or an alloy) or plastic that will be selected so that the resulting catheter will have characteristics of stiffness and bending that fall within the stated limits. Additional examples of ways to vary the stiffness and tensile strength include transverse breaks in a material, advance of the material so that it "doubles up," additional layers of the same or different material, tensioning or relaxing tension on the catheter, and applying electricity to a memory metal.

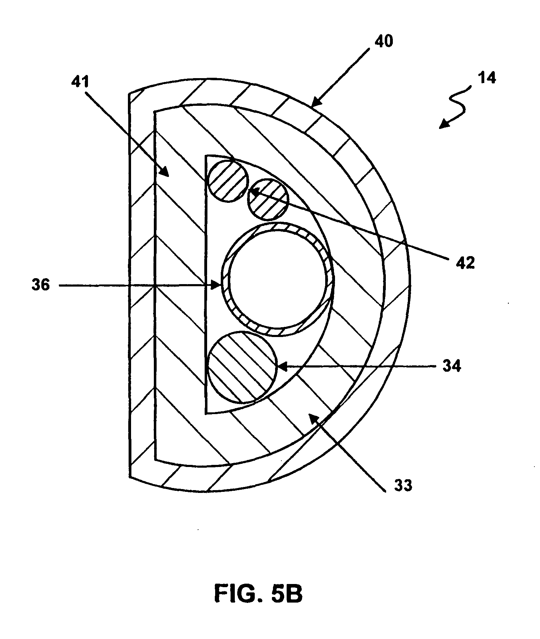

As illustrated in FIG. 5B, in some embodiments of an apparatus of the invention, guiding mandrel is combined with at least a portion of the catheter 14 to form a structure which provides the functions of both, a wall/mandrel 41. In this figure, the wall/mandrel 41 of catheter 14 can be varied in dimensions as described in the previous section of this specification directed to a separate mandrel, with the same resulting changes in function. For example, changing the thickness of the wall/mandrel 41 that functions as the mandrel portion changes the flexibility and preferred direction of bending of the catheter. In many cases, the wall/mandrel 41 will be thinner than other portions of the catheter wall 33 so that wall/mandrel 41 controls bending. Alternatively, wall/mandrel 41 can be formed of a different material than the other portions 33 of the catheter walls (i.e., one with a lower tensile and/or flexural strength) in order to facilitate bending.

Returning now to FIG. 5A, the guiding mandrel 32 is generally located in the interior of catheter 14, where it shares space with other functional elements of the catheter. For example and as shown in FIG. 5A, thermal energy delivery device lumen 34 can receive any of a variety of different couplings from an energy source 20 to a thermal energy delivery device (functional element) further along the catheter, including but not limited to a wire or other connector between thermal energy elements. Alternatively or concurrently, hollow lumen(s) 36 for delivery or removal of a fluid or solid connectors for application of a force to a mechanical element can be present, so no limitation should be placed on the types of energy, force, or material transporting elements present in the catheter. These are merely some of the possible alternative functional elements that can be included in the intradiscal portion of the catheter. Accordingly, a general description will now be given of some of the possible functional elements.

Since the purpose of the inventive catheter is to repair tears or fissures in a disc by operation of the instrument at the tear location adjacent to or inside the disc, a functional element is provided in or on the catheter to carry out that purpose.

Non-limiting examples of functional elements include any element capable of aiding diagnosis, delivering energy, or delivering or removing a material from a location adjacent the element's location in the catheter, such as an opening in the catheter for delivery of a fluid (e.g., dissolved collagen to seal the fissure) or for suction, a thermal energy delivery device (heat source), a mechanical grasping tool for removing or depositing a solid, a cutting tool (which includes all similar operations, such as puncturing), a sensor for measurement of a function (such as electrical resistance, temperature, or mechanical strength), or a functional element having a combination of these functions.

The functional element can be at varied locations in the intradiscal portion of the catheter, depending on its intended use. Multiple functional elements can be present, such as multiple functional elements of different types (e.g., a heat source and a temperature sensor) or multiple functional elements of the same type (e.g., multiple heat sources spaced along the intradiscal portion).

One of the possible functional elements present on intradiscal section 16 is a thermal energy delivery device 18. A variety of different types of thermal energy can be delivered including but not limited to resistive heat, radio frequency (RF), coherent and incoherent light, microwave, ultrasound and liquid thermal jet energies. In one embodiment, thermal energy delivery device 18 is positioned proximal to the distal portion of intradiscal section 16 so that there is no substantial delivery of energy at the distal portion, which can then perform other functions without being constrained by being required to provide energy (or resist the resulting heat).

Some embodiments (e.g., FIG. 5B) have an interior infusion lumen 36. Infusion lumen 36 is configured to transport a variety of different mediums including but not limited to electrolytic solutions (such as normal saline), contrast media (such as Conray meglumine iothalamate), pharmaceutical agents, disinfectants, filling or binding materials such as collagens or cements, chemonucleolytic agents and the like, from a reservoir exterior to the patient to a desired location within the interior of a disc (i.e., the fissure). Further, infusion lumen 36 can be used as an aspiration lumen to remove nucleus material or excess liquid or gas (naturally present, present as the result of a liquefying operation, or present because of prior introduction) from the interior of a disc. When used to transport a fluid for irrigation of the location within the disc where some action is taking place (such as ablation, which generates waste materials), the infusion lumen is sometimes referred to as an irrigation lumen. Infusion lumen 36 can be coupled to medium reservoir 21 through the catheter (see FIG. 3B).

Included in the particular embodiment shown in this figure is one or more sensor lumens 42. An example is a wire connecting a thermal sensor at a distal portion of the catheter to control elements attached to a connector in the proximal handle 11 of the catheter.

Also included in the embodiment shown in FIG. 5A is an optional energy directing device 43 including but not limited to a thermal reflector, an optical reflector, thermal insulator, or electrical insulator. Energy directing device 43 is configured to limit thermal and/or electromagnetic energy delivery to a selected site of the disc and to leave other sections of the disc substantially unaffected. Energy directing device 43 can be positioned on an exterior surface of catheter intradiscal section 16 and/or catheter 14 as well as in an internal portion of the catheter intradiscal section 16 and/or catheter 14. For example, the energy can be directed to the walls of the fissure to cauterize granulation tissue and to shrink the collagen component of the annulus, while the nucleus is shielded from excess heat.

In various embodiments, catheter intradiscal section 16 and/or distal portion 28 are positionable to selected site(s) around and/or adjacent to inner wall 22 of annulus fibrosus 122 for the delivery of therapeutic and/or diagnostic agents including but not limited to, electromagnetic energy, electrolytic solutions, contrast media, pharmaceutical agents, disinfectants, collagens, cements, chemonucleolytic agents and thermal energy. Intradiscal section 16 is navigational and can reach the posterior, the posterior lateral, the posterior medial, anterior lateral, and anterior medial regions of the annulus fibrosus, as well as selected section(s) on or adjacent to inner wall 22.

In one embodiment, intradiscal section 16 is positioned along the posterior lateral wall of the intervertebral disc, along the anterior lateral wall of the intervertebral disc, or along the anterior medial wall of the intervertebral disc, as it is steerable and can be positioned at any of the above-mentioned sites. In a preferred embodiment, intradiscal section 16 is positioned adjacent to the entire posterior wall of the disc. Sufficient thermal energy can then be delivered, for example, to selectively heat the posterior annulus to cauterize granulation tissue and shrink the collagen component of the wall around and adjacent to fissure 44 without undue damage to other portions of the intervertebral disc, particularly the nucleus. These actions help close the fissure in the annulus.

In one embodiment, intradiscal section 16 and/or distal portion 28 delivers electromagnetic energy to heat tissue and thereby reduce pain at a selected site, (e.g., to any portion of annulus fibrosis 122), especially along the posterior wall of the annulus fibrosis. The use of temperature-controlled electromagnetic heating, combined with the positional control of the catheter of the invention, allows preferential heating of selected location(s) within the disc.

In one embodiment, intradiscal section 16 and/or distal portion 28 delivers thermal energy to reduce pain at a selected site (e.g., any portion of annulus fibrosis 122), without ablation or removal of any disc material adjacent to intradiscal section 16 of catheter 14 and with or without removal of water vapor from the disc but without charring the nucleus. Intradiscal section 16 also can heat the collagen components of the annulus, thereby shrinking the annulus, with or without desiccating local tissue.

In one embodiment, intradiscal section 16 and/or distal portion 28 delivers RF energy to reduce pain at a selected site (e.g., to any portion of annulus fibrosis 24 and/or nucleus pulposis 26), especially along the posterior wall of the annulus fibrosis. Use of RF, with its potential for fine temperature control, combined with positional control of the catheter, allows exquisite local heating control within the disc.

In the preferred embodiment of FIG. 5A, markings 38 are visible on the portion of the catheter that is located during normal operation outside the body being acted upon, particularly for embodiments in which the proximal end of the catheter is designed to be directly manipulated by the hand of the physician. Advancement of the catheter into the introducer will advance the markings into the introducer, thereby showing how far the catheter has been advanced into the nucleus. Such a visible marking 38 can be positioned on an exterior surface of the catheter or can be present on an interior surface and visible through a transparent outer covering or sheath. Preferred are visible markings every centimeter to aid the physician in estimating the catheter tip advancement.

If desired, visible markings can also be used to show twisting motions of the catheter to indicate the orientation of the bending plane of the distal portion of the catheter. It is preferred, however, to indicate the distal bending plane by the shape and feel of the proximal end of the catheter assembly. The catheter can be attached to or shaped into a handle 11 that fits the hand of the physician and also indicates the orientation of the distal bending plane. Both the markings and the handle shape thus act as control elements to provide control over the orientation of the bending plane; other control elements, such as plungers or buttons that act on mechanical, hydrostatic, electrical, or other types of controls, can be present in more complex embodiments of the invention.

Additionally, a radiographically opaque marking device can be included in the distal portion of the catheter (such as in the tip or at spaced locations throughout the intradiscal portion) so that advancement and positioning of the intradiscal section can be directly observed by radiographic imaging. Such radiographically opaque markings are preferred when the intradiscal section is not clearly visible by radiographic imaging, such as when the majority of the catheter is made of plastic instead of metal. A radiographically opaque marking can be any of the known (or newly discovered) materials or devices with significant opacity. Examples include but are not limited to a steel mandrel sufficiently thick to be visible on fluoroscopy, a tantalum/polyurethane tip, a gold-plated tip, bands of platinum, stainless steel or gold, soldered spots of gold and polymeric materials with radiographically opaque filler such as barium sulfate. A resistive heating element or an RF electrode(s) may provide sufficient radio-opacity in some embodiments to serve as a marking device.

A sheath 40 can optionally be positioned around catheter 14. Sheath 40 provides a flexible surface that is smooth and provides for easy introduction into a selected area within the disc. Sheath 40 can be made of a variety of different materials including but not limited to polyester, rayon, polyimide, polyurethane, polyethylene, polyamide and silicone. When visible markings are present to indicate the advancement of the catheter, either the sheath carries the markings, or the sheath is clear to reveal markings underneath.

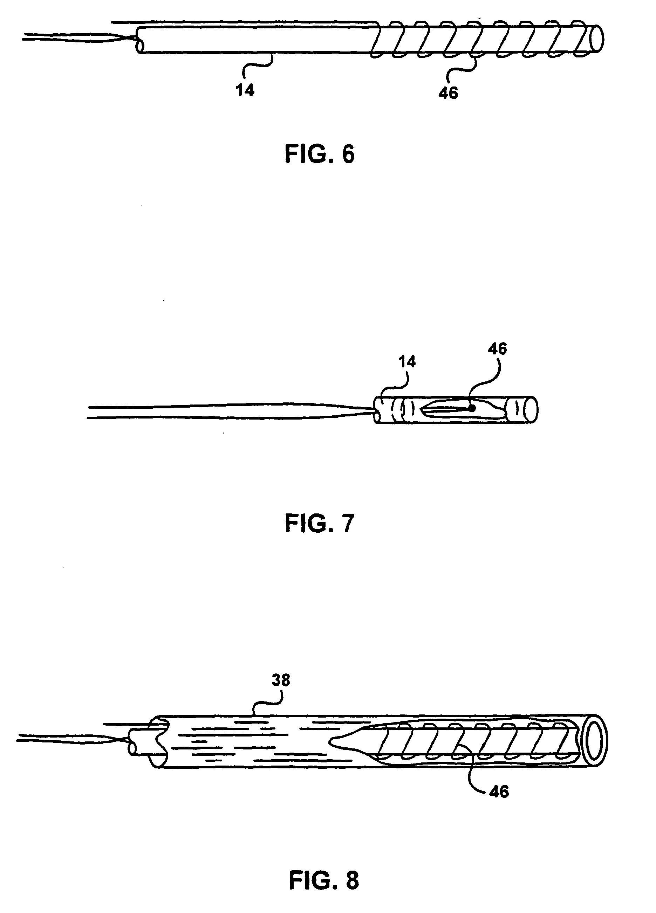

In one preferred embodiment, thermal energy delivery device 18 is a resistive heating device. The thermal energy delivery device 18 can be a band or a coil. As illustrated in FIG. 6, a heating coil 46 is positioned around an exterior of catheter 14 The heating element 46 need not be in the shape of a coil. For instance, the heating element can be in the form of a thin flexible circuit which is mountable on or in substantially one side of the intradiscal portion of the catheter. Heating element 46 is powered by a direct current source 20 (and less preferably a source of alternating current). Heating element is made of a material that acts as a resistor. Suitable materials include but are not limited to stainless steel, nickel/chrome alloys, platinum, and the like.

Preferably, the heating element is inside the intradiscal section of catheter 14 (FIG. 8). The resistive material is electrically insulated and substantially no current escapes into the body. With increasing levels of current, element 46 heats to greater temperature levels. Additionally, a circuit can be completed substantially entirely at intradiscal section 16 and a controllable delivery of thermal energy is achieved. In one embodiment, 2 watts pass through heating element 46 to produce a temperature of about 55 ° C in a selected target such as fissure 44, 3 watts produces 65°C, 4 watts produces 75°C, and so on.

In another embodiment, thermal energy delivery device 18 is a radio frequency electrode, such as a band or coil. As illustrated in FIG. 6, RF electrode 46 is positioned on an exterior of catheter 14. RF electrode 46 is powered by an RF generator. The electrode is made of suitable materials including but not limited to stainless steel or platinum. The RF electrode is located on intradiscal section of catheter 14. Increasing levels of current conducted into disc tissue heat that tissue to greater temperature levels. A circuit can be completed substantially entirely at intradiscal section 16 (bipolar device) or by use of a second electrode attached to another portion of the patient's body (monopolar device). In either case, a controllable delivery of RF energy is achieved.

In another embodiment sufficient energy is delivered to the intervertebral disc to heat and shrink the collagen component of the annulus but not ablate tissue adjacent to catheter 14. With a resistive heating device, the amount of thermal energy delivered to the tissue is a function of (i) the amount of current passing through heating element 46, (ii) the length, shape, and/or size of heating element 46, (iii) the resistive properties of heating element 46, (iv) the gauge of heating element 46, and (v) the use of cooling fluid to control temperature. All of these factors can be varied individually or in combination to provide the desired level of heat. Power supply 20 associated with heating element 46 may be battery based. Catheter 14 can be sterilized and may be disposable.

Referring now to FIG. 7, a thermal sensor 48 may be positioned in an interior location of catheter 14. In another embodiment, thermal sensor 48 is positioned on an exterior surface of catheter 14. A thermal sensor can be used to control the delivery of energy to thermal energy delivery device 18. A potting material can be used to fix the position of thermal sensor 48 and provide a larger area from which to average the measured temperature. Thermal sensor 48 is of conventional design, including but not limited to a thermistor; T type thermocouple with a copper-constantan junction; J type, E type, and K type thermocouples; fiber optics; resistive wires; IR detectors; and the like. Optionally, there may be a lumen 42 for the thermal sensor connection.

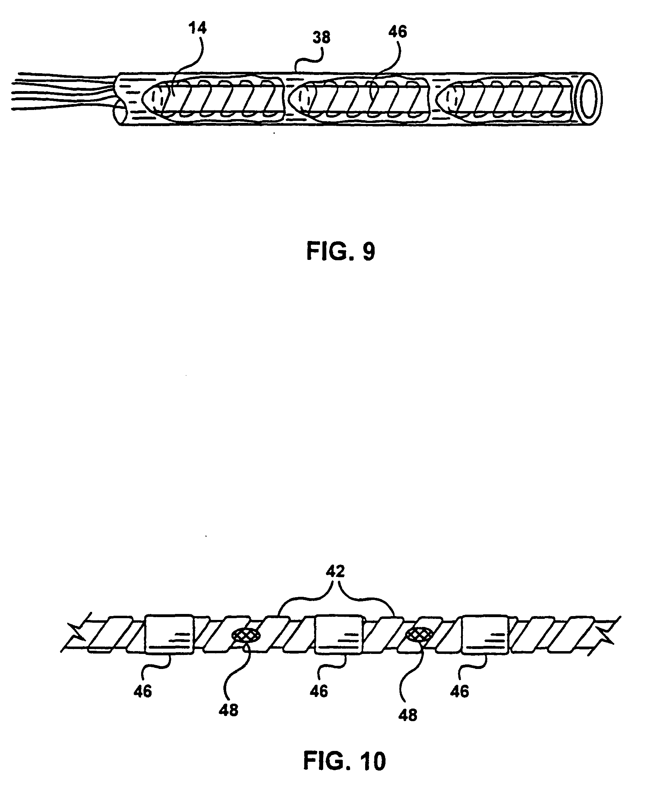

As illustrated in FIG. 8, sheath 40 may be used to cover resistive heating element 46. A plurality of resistive heating elements 46 can be used (FIG. 9) in a catheter of the invention.

Referring now to the embodiment shown in FIG. 10, thermal energy delivery device 18 comprises one or more resistive heating elements 46 coupled to a resistive heating energy source. Resistive heating elements 46 are positioned along intradiscal section 16 at locations where they controllably deliver thermal energy to selected structures, including granulation tissue in a fissure 44 and the annulus surrounding the fissure. Resistive heating elements 46 can be segmented and multiplexed so that only certain resistive heating elements, or combinations of resistive heating elements, are activated at any one particular time. Thermal sensor 48 can be positioned between resistive heating elements 46 and/or at an exterior or interior location of catheter 14. In the embodiment illustrated in FIG. 10, catheter 14 can be prepared with a wound helical structural element 42 to increase flexibility and minimize kinking. In this figure, multiple, spaced-apart structural elements 46 are shown which could have (for example) ports through which a material is delivered. However, other structures and geometries are suitable for catheter 14, including but not limited to a substantially smooth surface (and specifically including the devices using an internal guide mandrel as previously described). For example, a sheath can be provided over the heating element, and the guiding mandrel inside the coil can be encapsulated in silicone potting material. The tubing flexibility and the silicone potting material prevent kinking. Additionally, sheath 40 can be positioned around catheter 14 and also around resistive heating elements 46 to afford a substantially smooth surface. Resistive heating elements 46 can be at least partially covered by a thermally insulating material, for example, along one side of the catheter, to selectively heat disc tissue on the opposite side.

In another embodiment, there are two monopolar electrodes on the distal end of the RF probe. One electrode occupies a portion of the side of the distal end and the other electrode is the distal-most tip of the RF probe. The electrodes are operated independently to provide different tissue temperatures. In one configuration, the side electrode has a smaller area than the end electrode. If the side electrode receives the same power as the end electrode, it will provide more concentrated current and thus will produce more thermal energy (within a given unit volume). The end electrode will provide gentler, less concentrated current and will produce less thermal energy (within a given unit volume), for example, to shrink collagen in the annulus without denaturing said collagen.

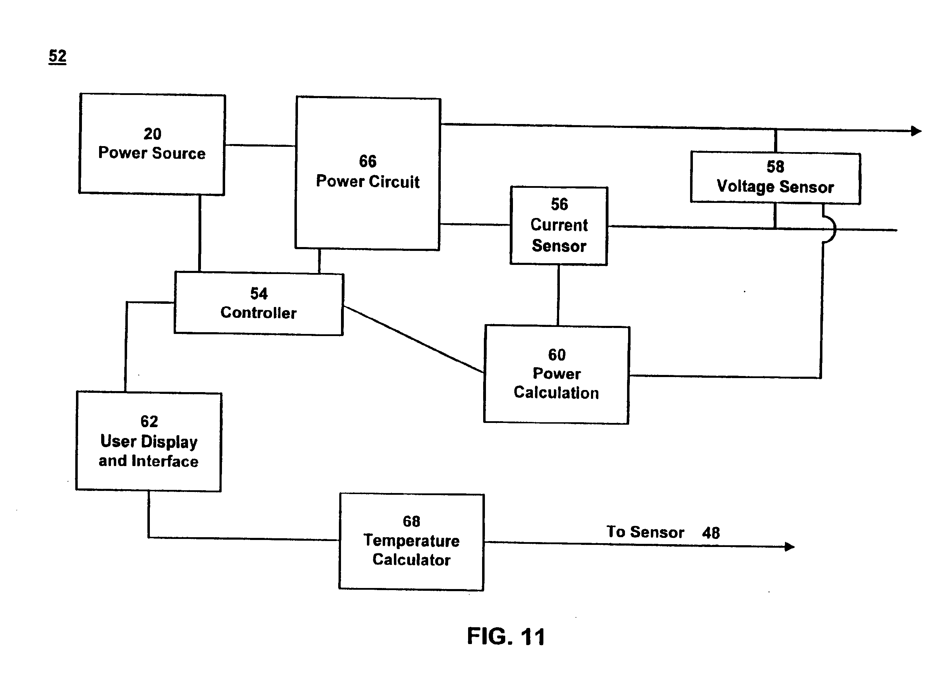

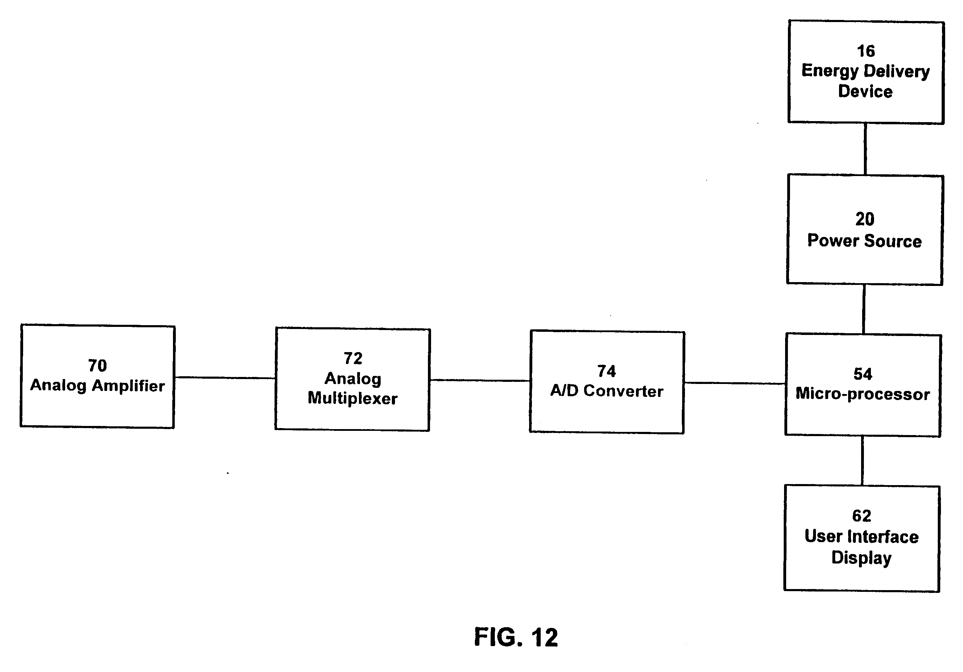

Referring now to FIGS. 11 and 12, an open or closed loop feedback system 52 couples sensors 48 to controller 54. As illustrated in FIG. 10, thermal energy delivery device 18 is a resistive heating element 46. It will be appreciated that the embodiments illustrated in FIGS. 10 and 11 are readily adaptable to other thermal energy delivery sources (e.g., for radio frequency energy, the resistive heating element is replaced with insulated RF probe(s) and the energy source is an RF generator).

The temperature of the tissue or of element 46 (FIG. 10) is monitored by sensors 48, and the output power of energy source 20 adjusted accordingly. The physician can, if desired, override control system 52. A microprocessor can be included and incorporated in the closed or open loop system to switch power on and off, as well as to modulate the power. The closed loop system utilizes a microprocessor to serve as a controller 54 which acts to monitor the temperature and adjust the power accordingly. Alternatives to the microprocessor are, for example, analog control circuitry and a logic controller.

With the use of sensors 48 and feedback control system 52, a tissue adjacent to resistive heating elements 46 can be maintained at a desired temperature for a selected period of time without aberrant high temperature fluctuations. Each resistive heating element 46 can be connected to separate control and power supply resources, which generate an independent output for each resistive heating element 46. For example, a desired thermal output can be achieved by maintaining a selected energy at resistive heating elements 46 for a selected length of time.

When a resistive heating element 46 is used, current delivered through resistive heating element 46 can be measured by current sensor 56. Alternatively, when an RF electrode is used, the current delivered through the electrode can also be measured by the current sensor 56. Voltage can be measured by voltage sensor 58. Resistance and power are then calculated at power calculation device 60. These values can then be displayed at user interface and display 62. Signals representative of power and resistance values are received by a controller 54.

A control signal is generated by controller 54 that is related to the current and voltages. The control signal is used by power circuits 66 to adjust the power output in an appropriate amount in order to maintain the desired temperature delivered at respective resistive heating elements 46.

In a similar manner, temperatures detected at sensors 48 provide feedback for maintaining a selected power. The actual temperatures are measured at temperature measurement device 68, and the temperatures are displayed at user interface and display 62. A control signal is generated by controller 54 that is related to the actually measured temperature and a desired temperature. The control signal is used by power circuits 66 to adjust the power output in an appropriate amount in order to maintain the desired temperature delivered at the respective sensor 48. A multiplexer can be included to measure current, voltage, and temperature at the sensors 48, 56 and 58, so that appropriate energy can be delivered to resistive heating elements 46.

Controller 54 can be a digital or analog controller or a computer with software. When controller 54 is a computer, it can include a CPU coupled through a system bus. Included in this system can be a keyboard, a disc drive or other non-volatile memory system, a display, and other peripherals, as are known in the art. Also coupled to the bus can be a program memory and a data memory.

User interface and display 62 includes operator controls and a display. Controller 54 can be coupled to imaging systems well known in the art.

The output of current sensor 56 and voltage sensor 58 is used by controller 54 to maintain a selected power level at resistive heating elements 46. A predetermined profile of power, temperature or energy to be delivered can be incorporated in controller 54.

Circuitry, software, and feedback to controller 54 result in process control and in the maintenance of the selected power that is independent of changes in voltage or current. Control can include (i) the selected power and (ii) the duty cycle (wattage and on-off times). These process variables are controlled and varied while maintaining the desired delivery of power independent of changes in voltage or current, based on temperatures monitored at sensors 48.

In the embodiment shown in FIG. 12, current sensor 56 and voltage sensor 58 are connected to the input of an analog amplifier 70. Analog amplifier 70 can be a conventional differential amplifier circuit for use with sensors 48, 56 and 58. The output of analog amplifier 70 is sequentially connected by an analog multiplexer 72 to the input of A/D converter 74. The output of analog amplifier 70 is a voltage which represents the respective sensed parameters. Digitized amplifier output voltages are supplied by A/D converter 74 to microprocessor 54. Microprocessor 54 may be a type 68HCII available from Motorola. However, it will be appreciated that any suitable microprocessor or general purpose digital or analog computer can be used for the parameters of temperature, voltage or current.

Microprocessor 54 sequentially receives and stores digital representations of temperature. Each digital value received by microprocessor 54 corresponds to different parameters.

Calculated power and temperature values can be indicated on user interface and display 62. Alternatively, or in addition to the numerical indication of power, calculated power values can be compared by microprocessor 54 with power limits. When the values exceed predetermined power or temperature values, a warning can be given on user interface and display 62, and additionally, the delivery of electromagnetic energy can be reduced, modified or interrupted. A control signal from microprocessor 54 can modify the power level supplied by energy source 20.

In a preferred embodiment of the invention, the materials that make up the various parts of an apparatus of the invention have the following characteristics:

| Component | Tensile Strength in MPa | % Elongation | Conductivity cal/cm²/cm/ sec/°C | Resistivity nΩ*m | Melt temp. °C | Geometry (height, width, and/or dia.) in mm |
|---|---|---|---|---|---|---|
| Mandrel | 600-2000 | 5-100 | N/A | N/A | N/A | height 0.2-2.3 width 0.05-0.5 |
| Heating Element | 300 min. | 20 (min.) | .025-0.2 | 500-1500* | N/A | 0.05-0.5 dia. |
| Conductor Wire | N/A | N/A | 2-10 | 150 max * | N/A | 0.1-0.5 dia. |
| Plastic Sheath | N/A | 25 (min.) | N/A | N/A | 80° (min.) | 0.05-0.2 thickness |

Another preferred characteristic is that the minimum ratio of heating element resistivity to conductor wire resistivity is 6: 1; the preferred minimum ratio of guiding mandrel height to guiding mandrel width is 2:1. Tensile strength and % elongation can be measured according to ASTME8 (tension test of metallic materials). Conductivity and resistivity can be determined by procedures to be found in ASTM Vol. 2.03 for electrothermal properties. The guidable apparatus described herein can be used in methods to treat local areas within a disc.

A particularly preferred embodiment of a catheter of the invention can be prepared using a covering sheath of polyimide with an outside diameter of 1 mm and a wall thickness of 0.05 mm. Such a sheath provides a significant fraction of the stiffness and torsional strength appropriate for the catheter. Internal to the polyimide sheath and in the intradiscal section of the catheter is a heating coil of insulated nickel/chromium wire that has an outside diameter that matches the interior diameter of the polyimide sheath. This heating coil provides both heat and additional stiffness to the assembly. Also internal to the polyimide sheath on each side of the coil (longitudinally) is a 0.1 mm-walled, 304 stainless-steel, metallic band whose outer diameter matches the inner diameter of the sheath, the distal band having a hemispherical end that exits the end of the polyimide sheath and creates a blunt tip 29 at the end of the catheter. These bands provide enhanced radio-opacity for fluoroscopic visualization, as well as some of the stiffness of the assembled apparatus. Proximal to the proximal metallic band and internal to the sheath is an additional polyimide tube 47 that increases the stiffness of the catheter in the region that transitions from the intradiscal section containing the coil to the rigid proximal section. Proximal to the second polyimide tube and internal to the sheath is a 304 stainless steel (fully hard) hypodermic tube with an outside diameter matching the inside diameter of the polyimide sheath and a wall thickness of 0.1 mm. This combination provides the rigidity needed for a physician to advance the distal portion of the device inside a nucleus pulposus and provides tactile force feedback from the tip to the physician.

In some embodiments, inside the bands, coil, hypodermic tube, and both the polyimide sheath and internal polyimide tube is a guiding mandrel that extends from a proximal handle to the catheter tip. In one embodiment, this mandrel is 0.15 mm by 0.5 mm and formed from 304 stainless steel. In another embodiment, it is a 0.3 mm diameter 304 stainless steel wire, with the distal 2.5 cm flattened to 0.2 mm by 0.5 mm.

Inside the center of the heating coil is a T-type thermocouple potted with cyanoacrylate adhesive into a polyimide sheath and located alongside the mandrel. The thermocouple wire travels through the coil and hypodermic tube to the handle at the proximal end of the apparatus. Two copper conductor wires (36 gauge insulated with polyimide) are soldered to the heating coil and pass through the hypodermic tube and handle to the proximal handle's electrical connector, which allows a power supply and feedback controls to be connected to electrical elements in the catheter. One embodiment has the handle fitted with a 1-3 meter cable extension ending in an electrical connector to eliminate the need for a connector in the handle. This design reduces weight (from connector elements) on the proximal end and increases the physician's tactile feedback during device manipulation.

The entire inside of the catheter in one embodiment is encapsulated with a silicone material which removes air (which would insulate the heat created by the heating coil) and helps support the polyimide sheath to prevent collapse (i.e., increases stiffness). Instead of the silicone, another embodiment uses an epoxy which remains flexible after curing. Strain relief is provided between the catheter body and the handle with an elastomeric boot. The distal end of the catheter is pre-bent 15-30° off the longitudinal axis of the catheter at about 5-10 mm from the distal tip.

The catheter in one embodiment carries visible markings 38 on the hypodermic tube (with the markings being visible through the polyimide sheath) to indicate distance of insertion of the catheter into an introducer and/or distance that the distal end of the catheter extends out of the introducer into a disc. The catheter is also marked both visually and with tactile relief on its handle to indicate the direction of bending of the pre-bent tip and biased stiffness.

The guidable apparatus described herein can be used in any of a number of methods to treat annular fissures. Specific methods that can be carried out with an apparatus of the invention will now be described.

Discs with fissures can be treated non-destructively with or without the removal of nucleus tissue other than limited desiccation of the nucleus pulposus which reduces its water content. Degenerative discs with tears can also be treated. Fissures can also be ameliorated by shrinking the collagen component of the surrounding annulus to bring the sides closer to their normal position. Thermal shrinkage of collagen also facilitates ingrowth of collagen which increases annular stiffness. Fissures can also be repaired with sealants such as a filler (non-adhesive material that blocks the opening) and/or bonding material (adhesives or cements) which help seal the tear. The fissure can also be treated with global heating of the disc. Most of the heat will be directed toward the fissure, but the remainder of the disc will also receive some heat.

Degenerative discs with circumferential bulges can be treated non-destructively without desiccation or charring of tissues. Such bulges can be ameliorated by shrinking the collagen component of the annulus. To do that, the disc adjacent to the fissure is typically to a temperature in the range of about 45-70°C. Thermal shrinkage of collagen also facilitates ingrowth of more collagen, which further increases annular stiffness. If necessary, the area of the bulge can be reinforced with filler (non-adhesive material that blocks the opening), bonding material (adhesives) or other such sealants.

In some methods of the invention, filler and/or sealants and/or bonding materials such as collagen, albumin, and a mixture of fibrinogen and thrombin (fibrogen cements) are delivered to the fissure. Collagen from a variety of sources can be used (e.g., bovine extracted collagen from Semex Medical, Frazer PA, or human recombinant collagen from Collagen Corp., Palo Alto, CA). The collagen is injected dissolved or as a fine slurry, after which it gradually thickens (or may be heated) in the fissure, where the injected collagen provides a matrix for collagen disposition by the body.

A variety of different materials can also be delivered to the fissure, including but not limited to electrolyte and/or irrigating solutions (i.e., normal saline 5% glucose, etc.), contrast media (e.g., Conray meglumine iothalamate), pharmaceutical agents (such as the steroid methylprednisolone sodium succinate available from Pharmacia & Upjohn, Kalamazoo, MI, and nonsteroidal anti-inflammatory drugs), chemonucleolytic enzyme (e.g., chymopapain), hydrogel (such as disclosed in U.S. Patent No. 4,478,822), osteoinductive substances (e.g., BMP, see U.S. Patent No. 5,364,839), chondrocyte inductive substance (e.g., TGF-β) and the like. The materials are delivered via the catheter and/or introducer to the disc. Preferably, however, when precision placement of the material (as in a fissure) is necessary or desired, the delivery method uses the apparatus described above, especially when delivery to the posterior, posterior lateral, or posterior medial region of the disc is desired. The materials may be administered simultaneously or sequentially, such as beginning with an electrolytic solution (which helps the physician view the pathology) and following with products to seal a fissure.

The materials are delivered in an amount sufficient to decrease the extent of the fissure at least partially, preferably to fill the fissure completely. The delivered material can be fixed in position with an adhesive, with a hydrogel that is liquid at room temperature but gels at body temperature, with naturally occurring processes (such as interaction of fibrinogen and thrombin) within the disc, or by heating the disc as described in more detail below.

To seal a fissure or tear, a combination of thrombin and fibrinogen is injected at the fissure, after which it coagulates and forms a seal over the fissure. A kit with appropriate syringes and other equipment is available from Micromedics, Inc., Eagan, MN. Frozen fibrinogen solution is thawed in its plastic bag and then dispensed to a small med cup. Thrombin is reconstituted with sterile water in the "slow gel" concentration (100 units/ml) for tissue bonding. For example, 100 ml is added to a vial containing 10,000 units. Thrombin solution is withdrawn from the vial and dispensed to a second med cup. The two syringes are filled equally, one with each solution. Then the syringe tips are each twisted into an applicator that mixes the solutions before passing them to an administration tube. The syringes are fitted into the dual syringe holder and the plunger link, which helps the practitioner administer equal amounts of thrombin and fibrinogen. Then the practitioner connects the administration tube to the proximal end of the inventive catheter, depresses the plungers and dispenses the sealant solution to the fissure. The thrombin and fibrinogen react and form a natural seal over the fissure.

Chymopapain can be injected through the subject catheter, particularly near a herniation of the disc. This may be desired because the intradiscal pressure is excessive. Chymopapain splits side chains off proteoglycan molecules, thereby decreasing their ability to hold water and their volume. The disc gradually loses water and decreases in size. A typical dose is 0.75 to 1.0 ml (2000 pKat/ml).

In some embodiments, thermal energy (e.g., RF or resistive) is delivered to a selected section of the disc in an amount that does not create a destructive lesion to the disc, other than at most a change in the water content of the nucleus pulposus. In one embodiment there is no removal and/or vaporization of disc material positioned adjacent to an energy delivery device positioned in a nucleus pulposus. Sufficient thermal energy is delivered to the disc to change its biochemical and/or biomechanical properties without structural degradation of tissue.

Neurophysiologic modifications include denervation of pain receptors in a fissure in the annulus fibrosis and inactivation of neurotransmitters. Heat-sensitive enzymes also are inactivated.

Degenerative intervertebral discs with fissures are treated by denervating nerves embedded in the anterior wall of the annulus fibrosis as well as nerves in and outside the annulus. Electromagnetic energy is used to ablate granulation tissue which is pain sensitive and forms the annulus fibrosis wall.

Heat produced by RF electromagnetic energy is also used to break down selected enzyme systems and neurotransmitters that generate pain within the disc. Generally, these enzymes (such as cytokines, phospholipase A2 and prostaglandin E2) and neurotransmitters only work within small ranges of pH and temperature. Therefore, the application of heat inactivates some of these proteins.

Electromagnetic energy is applied to heat and shrink the collagen component of the annulus fibrosis. This reduces the redundancy in the disc roll that is created in a degenerative disc. This also reduces a "pushing out" effect that created a contained herniation. The tightening and stiffening of the annulus fibrosis helps the disc function more normally. Tightening the annulus fibrosis may help stabilize the spine and relieve pain. Careful application of electromagnetic energy locally increases the stiffness of the disc in appropriate locations without overheating and harming other parts of the disc.

Resistive heating is delivered to a selected section of the disc in an amount which does not create the destructive legion to the disc, other than at most a change in the water content of the nucleus pulposis. In one embodiment, there is no removal and/or vaporization of disc material positioned adjacent to the resistive heater in a nucleus pulposis. Sufficient thermal energy is delivered to change to the discs biochemical and/or biomechanical properties without structural degradation of tissue. Neurophysiologic modifications include denervation of nociceptores in a tear or fissure in the annulus fibrosis and inactivation of neurotransmitters. Heat-sensitive enzymes also are inactivated.

Degenerative intervertebral discs with fissures are treated by denervating nerves embedded in the interior wall of the annulus fibrosis as well as nerves in the annulus and its exterior surface. Resistive heating is used to selective cauterize granulation tissue which is pain sensitive and forms in a tear or fissure.

Controlled thermal energy also breaks down heat-sensitive enzyme systems and neurotransmitters that generate pain both within the disc and external to the disc when they migrate out through a fissure. Generally, these enzymes and neurotransmitters only work within small ranges of pH and temperature at or near normal pH and temperature. Therefore, the application of heat inactivates some of these proteins.

Controlled thermal energy is applied to heat and shrink the collagen component of the annulus fibrosis. This reduces the redundancy in the disc roll that is created in a degenerative disc. Delivery of heat to the nucleus pulposis may optionally be used to remove some water (i.e., desiccate the nucleus) and permit the nucleus pulposis to withdraw. This reduces a "pushing out" effect that created a contained herniation. The combination of shrinking on the nucleus pulposis by desiccation and tightening up the annulus fibrosis wall rejuvenates the disc. Reducing the pressure in the disc and tightening the annulus fibrosis helps stabilize the spine and relieve pain. Application of controlled heat locally increases the stiffness of the disc in that local, which degree of stiffness can be selected to advantage using the fine temperature and positional control afforded by the present invention.

Thermal energy is used to cauterize granulation tissue which is pain sensitive and forms in a long-standing tear or fissure. Additionally or alternatively, thermal energy is used to seal at least a part of the fissure. To do that, the disc material adjacent to the fissure is typically heated to a temperature in the range of 45-70°C which is sufficient to shrink and weld collagen. This temperature is sufficient to shrink the collagen component of the annulus and strengthen the annulus. If the roll is fully circumferential, heat can be applied at successive locations around the circumference. Otherwise, the catheter is positioned and heat is applied to particular areas in need of treatment. In one method, tissue is heated to a temperature of at least 50°C for times of approximately one, two, three minutes, or longer, as needed to shrink the tissue back into place.

Delivery of thermal energy to the nucleus pulposus removes some water and permits the nucleus pulposus to shrink. This reduces a "pushing out" effect that may have contributed to the fissure. Reducing the pressure in the disc and repairing the fissure may help stabilize the spine and reduce pain.

Global heating of the disc also can be used to cauterize the granulation tissue and seal the fissure. In this embodiment of the method, the heating element is positioned away from the annulus but energy radiates to the annulus to raise the temperature of the tissue around the fissure. This global heating method can help seal a large area or multiple fissures simultaneously.

### EXAMPLES

Specific embodiments of the present invention will now be further described by the following, nonlimiting examples which will serve to illustrate in some detail various features of significance. The examples are intended merely to facilitate an understanding of ways in which the present invention may be practiced and to further enable those of skill in the art to practice the present invention. Accordingly, the examples should not be construed as limiting the scope of the present invention.

### Example 1

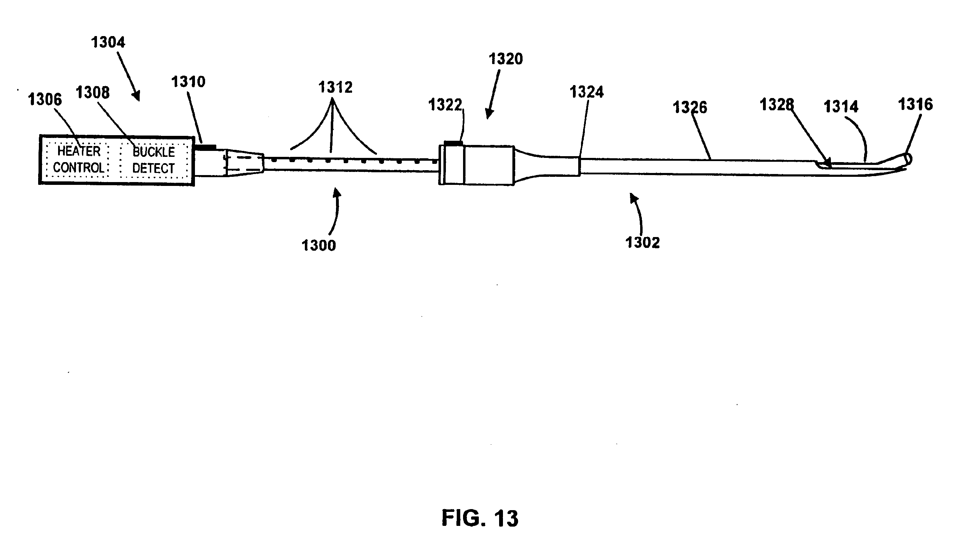

FIG. 13 shows a catheter 1300 and cannula 1302. Catheter 1300 comprises a handle 1304, an orientation indicator 1310, a stem 1312, a heater portion 1314 and a tip 1316. Handle 1304 includes buckle detector 1308 and heater delivery and control circuitry 1306. The catheter is shown partially within a tubular cannula 1302. The tubular cannula includes a hub portion 1320, a stem 1326 an a tip 1328. The hub includes a visual orientation indicator 1322 and a stem mount 1324.

Indicator 1310 on the handle 1304 of the catheter 1300 indicates the direction of curvature of tip 1316 as well as the preferred axis of deflection of the heater 1314 portion of the catheter. Visual depth indicators on the stem 1312 of the catheter 1300 indicate the extent to which the catheter protrudes from the tip 1328 of the cannula 1302. Visual indicator 1322 on the hub 1320 of cannula 1302 indicates the orientation of the opening in the tip 1328 of the cannula 1302. It is this opening on the tip 1328 of the cannula from which the catheter extends within the surgical site.

The catheter has sufficient column strength to be advanceable through a nucleus pulposus of an intervertebral disc and to be navigable along an inner wall of an annulus fibrosus. It can be advantageous for the catheter to include an intradiscal section that has a low enough flexural strength in an axial plane of the intervertebral disc, and a sufficient flexural strength in oblique and cephalo-caudal planes to be constrained within the axial plane of the intervertebral disc.

### Example 2

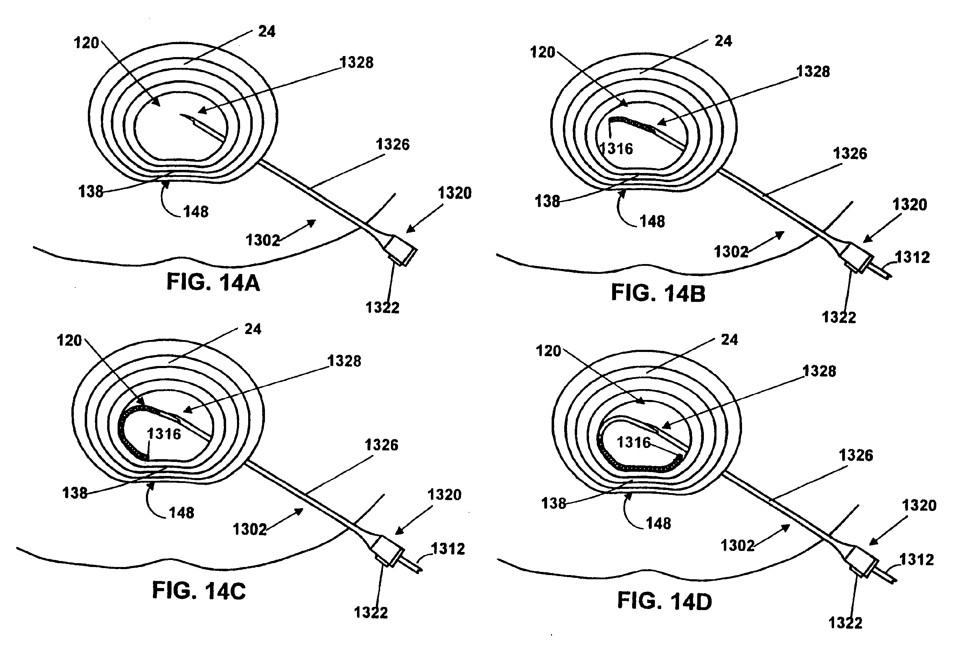

FIGS. 14A-D show a sequence of operations corresponding to the therapeutic delivery of heat to the nucleus pulposus 120 of a spinal disc. In FIG. 14A the cannula/introducer 1302 is introduced through a patient's back along a path which intercepts a selected disc. The insertion of the cannula into the appropriate location may be accomplished with x-ray imaging which allows visualization of the cannula tip 1328 with respect to the selected spinal disc. The tip of the cannula pierces the disc and extends the desired distance into the nucleus pulposus 120. Visual indicator 1322 on hub 1320 of the cannula shows the orientation of the opening in the tip 1328.

In FIG. 14B the catheter is inserted through the hub 1320 of the cannula 1302. Depth markings on the stem 1312 of the catheter indicate to the surgeon the point at which the tip 1316 and heater portions 1314 of the catheter have reached the nucleus pulposus.

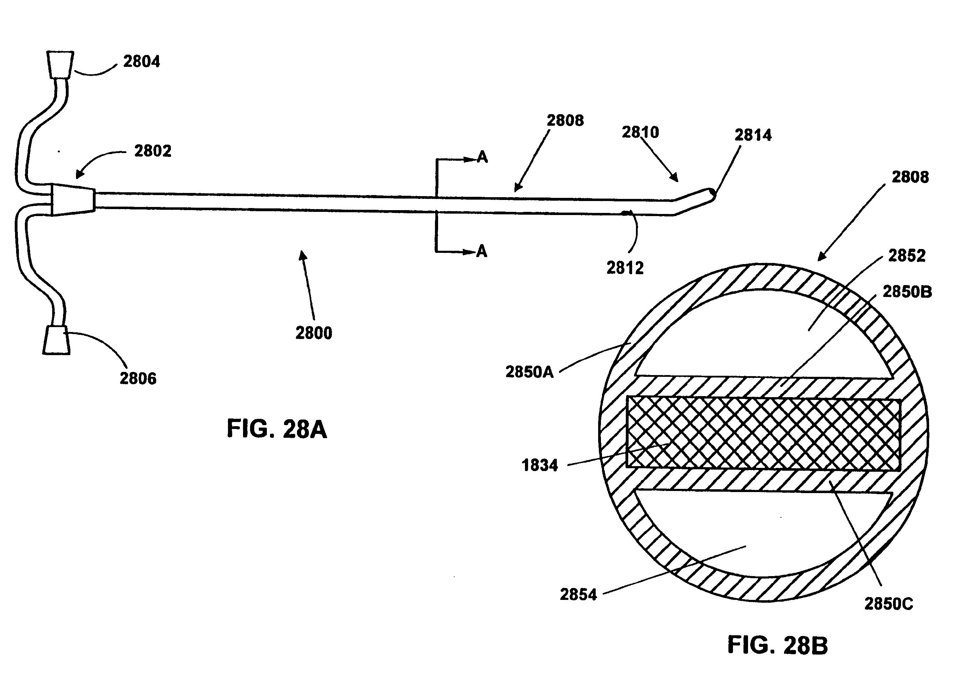

In FIG. 14C and D the insertion of the catheter continues causing the tip 1316 to traverse the interior walls of the annulus fibrosus placing the heated end portion of the catheter in contact with an arcuate portion of the interior wall of the disc. In this orientation heat can be delivered to a specific surgical site, i.e. a fissure or rupture. In alternate embodiments shown in FIGS. 28-29 material may be delivered to and/or removed from the areas of the nucleus pulposus 120 in contact with the catheter.

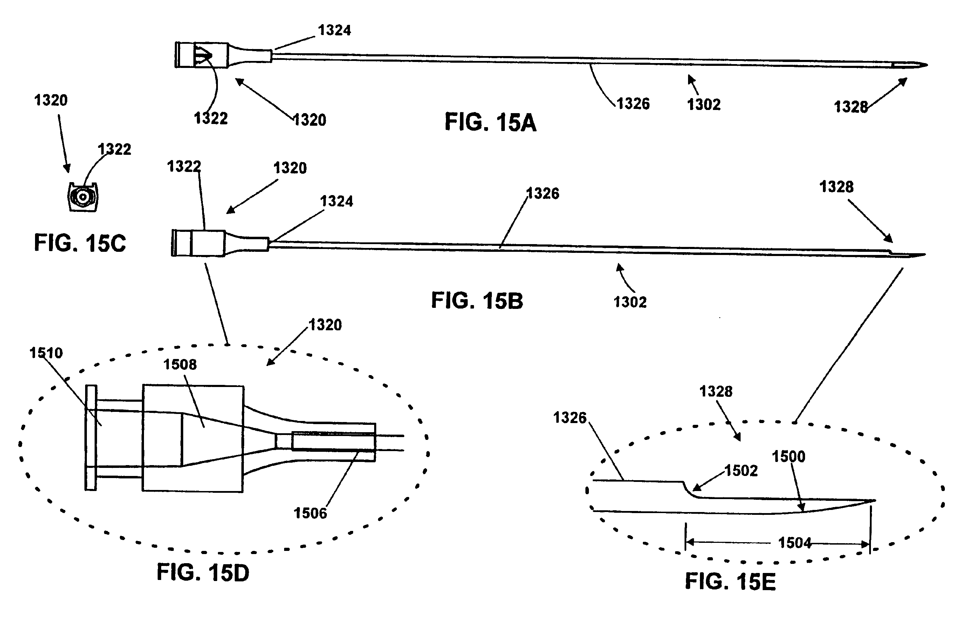

FIGS. 15A-E are detailed views of the cannula 1302. FIG. 15A shows a top view of the cannula with the needle opening at the tip 1328 aligned with visual needle opening indicator 1322 which is part of hub 1320. FIG. 15B shows a side view of cannula 1302. FIG. 15C shows an end view of hub 1320. A tactile indication of the orientation of the needle opening on the tip 1328 is provided by a recessed portion of the hub in which the visual indicator 1322 resides. FIG. 15D shows a cross-sectional view of the interior of hub 1320. The hub is tubular in cross-section and has three interior sections. The first section 1510 has a large diameter opening into which the catheter is introduced. In a preferred embodiment the opening is .175 inches inside diameter and has a gradually tapering length of 3.3 inches. This geometry is such that it can accomodate a taperer luer from a syringe. The next section 1508 is of equivalent length, i.e. .29 inches and tapers over its length to a diameter equal to the inside diameter of the cannula stem 1326. In an embodiment the cannula stem has a .058 outside diameter and a .0475 inside diameter. The remaining portion of the hub 1506 has a inside diameter of 044 inches. And an outside diameter of .145 inches. The stem 1326 may be press fit or adhesively or thermally bonded within section 1506 of the cannula hub 1320. The stem is preferably fabricated from stainless steel. Other biocompatible materials such as platinum are also a suitable material for the cannula. The cannula must be rigid enough to avoid buckling when the tip 1328 is inserted into the surgical site. The relatively long length of the cannula stem 1326, i.e. six inches requires that the cannula stem 1326 be fabricated from a rigid material.

FIG. 15E shows the details of the tip 1328 of the cannula. The tip has an opening 1504 which is approximately .275 inches in length. The opening is on one side of the stem 1326. The interior surface of the cannula tip is curved on an .75 inch radius This curvature initiates the bending of the catheter as it is extended through the opening in the cannula tip 1328. In an embodiment the opening extends vertically from one side of the stem to approximately the midpoint of the stem over a distance of approximately .029 inches. As discussed above the length of the opening along the cannula stem midsection is approximately .275 inches. At the end of the opening furthest from the tip of the cannula all interior surfaces of the opening are radiused in order not to abrade the catheter as it is inserted and retracted from the cannula.

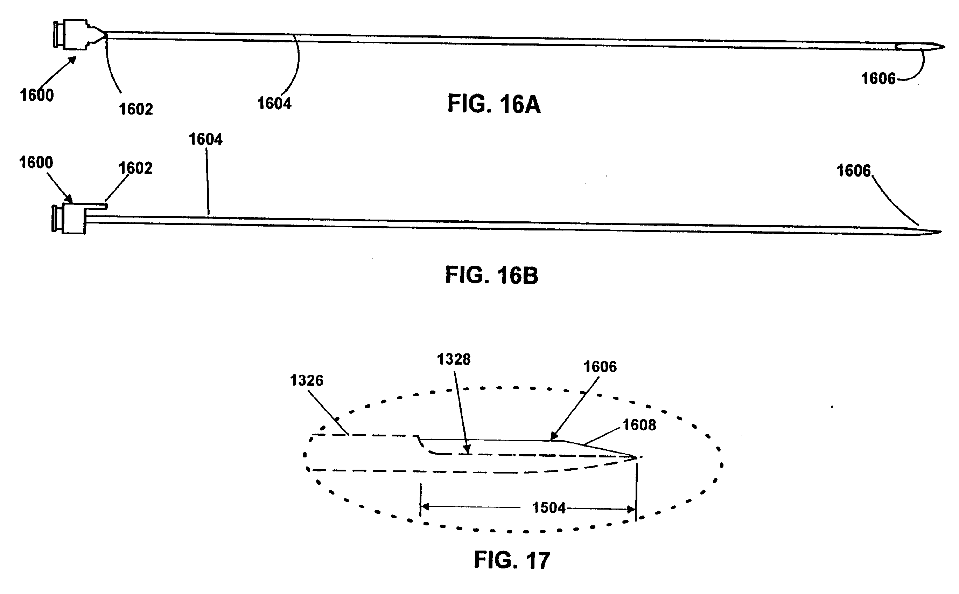

FIG. 16A-B and 17 show a stylette which is inserted into the cannula prior to insertion of the cannula into the surgical site. The stylette fills the tubular core of the cannula preventing material contamination of the cannula during insertion into the surgical site. The stylette 1600 includes a orientation indicator 1602, a stem 1604 and a tip 1606. In operation the stylette is inserted into the cannula and the orientation indicator 1602 locks into the recess 1322 (see FIG. 15C) of the cannula hub 1320. This causes the tip of the stylette to be aligned with the tip of the cannula.

FIG. 17 shows the stylette and cannula tips. The cannula stem 1326 and tip 1328 are shown in phantom line weight. The stylette tip 1606 has a sloped outer surface which complements the radius on the tip of the cannula. Thus the stylette and cannula form a needle point for piercing through the body to the nucleus pulposus in a spinal disc. When the cannula and stylette have been inserted into the surgical site the stylette is removed.

### Example 3

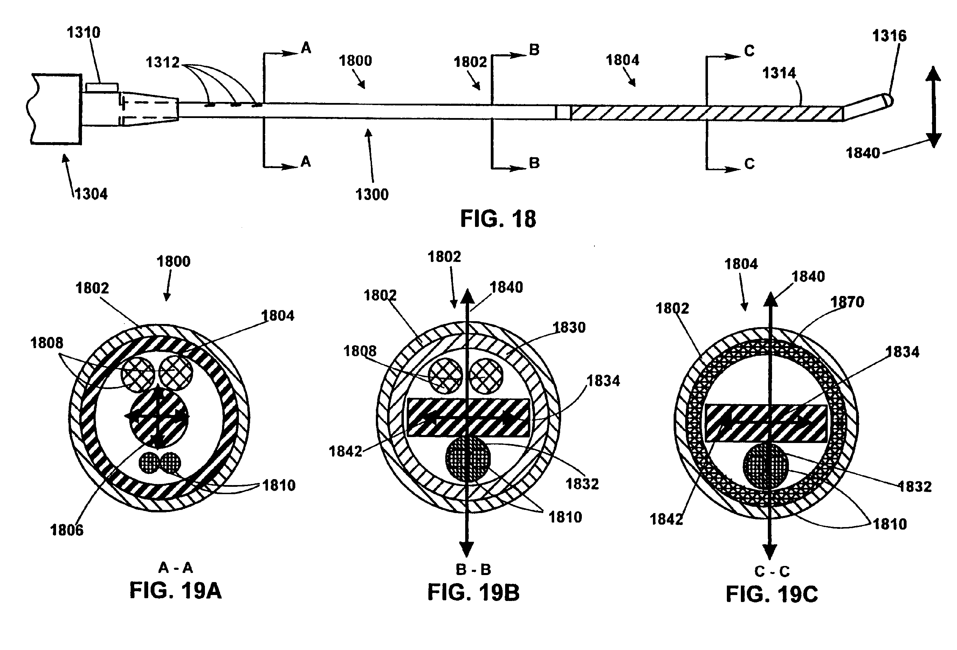

FIG. 18 is a detailed diagram of the catheter 1300 shown in FIG. 13. The catheter includes a handle 1304, a visual orientation indicator 1310, a stem 1312 with depth indicators, a heater portion 1314 and a tip 1316. The stem is divided into a proximal section 1800, a transition section 1802 and a distal-heater section 1804. The catheter is in an embodiment approximately 12 inches from handle 1304 to tip 1316. The catheter is No. 3 French (.038 inch) diameter. The heater section is two inches long starting from the tip.

FIGS. 19A-C show cross-sectional views at respectively the proximal section, the transition section and the distal heater section shown in FIG. 18. FIG. 19A is a cross-sectional view of the proximal section A-A shown in FIG. 18. The proximal cross-section 1800 includes an outer polyimide sheath 1802, a stainless steel tube 1804, a stainless steel core 1806, thermocouple conductors 1810, and electrical conductors 1808. The proximal section has no preferred bending axis since its moment of inertia is determined primarily by the rigid stainless steel tube and core members 1804 and 1806. Thus the proximal section of the catheter resists both bending and buckling as the catheter is inserted into the lumen.

FIG. 19B shows the transition cross-section 1802 of the catheter 1300. This view is taken along lines B-B shown in FIG. 18. The stainless steel tube found in FIG. 19A has been replaced with a relatively flexible polyimide tube 1830. This is housed within outer polyimide sheath 1802. Both of these tubes are flexible. Nevertheless the transition section has a preferred bending axis 1840. Which is orthogonal to the longitudinal axis 1842 of the stainless steel cross-member 1834. This cross-member provides the needed resistance to deflection along axis 1842 while allowing deflection along axis 1840.

FIG. 19C is a cross-section taken at the distal heater section of the catheter about C-C in FIG. 18. Outer polyimide sheath 1802 wraps around a resistive heating coil 1870. In the interior of the catheter a stainless steel cross-member 1842 provides the needed resistance to deflection along axis 1842. Axis 1842 in an embodiment coincides with the plane of the opening in the tip 1328 of the cannula 1302. Thus as the heater is extended through the opening in the cannula the axis on which it deflects 1840 is orthogonal to the plane of the opening. Thus if the plane of the opening is parallel to the interior side walls of the spinal disc the heater can be extended into the nucleus pulposus 120 (see FIGS. 14A-D) without contacting the bony members above or below each disc.

### Example 4

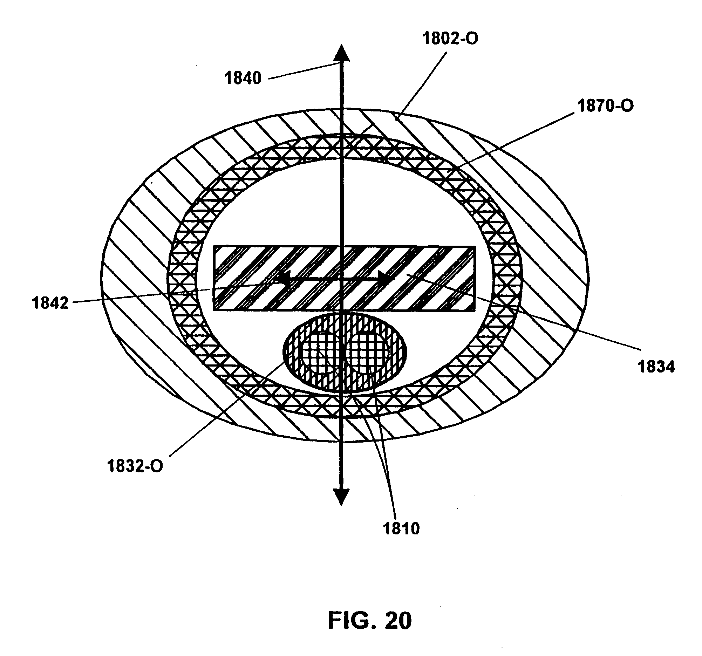

FIG. 20 shows a cross-sectional view of the distal-heater section of catheter 1300. The catheter in this embodiment is elliptical in shape. This particular configuration has two benefits not found in the above discussed embodiments. First, the elliptical shape of both the outer polyamide sheath 1802-0 and the heater coil 1870-0 enhances the resistance to deflection along the major axis of the ellipse which coincides with the longitudinal axis 1842 of the stainless steel. The deflection along axis 1840 is enhanced by aligning the minor axis of the ellipse parallel to deflection axis 1840. The additional benefit of the elliptical shape is that when both the catheter and cannula lumen have complementary elliptical shapes the catheter aligns itself within the cannula lumen with the major axis of both parts aligned. Thus it is not necessary for the physician to monitor visual indicator 1310 (see FIG. 18) since the catheter 1300 is slidably held within cannula 1302 in an arcuate orientation in which the major elliptical axes of both the cannula and catheter are parallel.

### Example 5

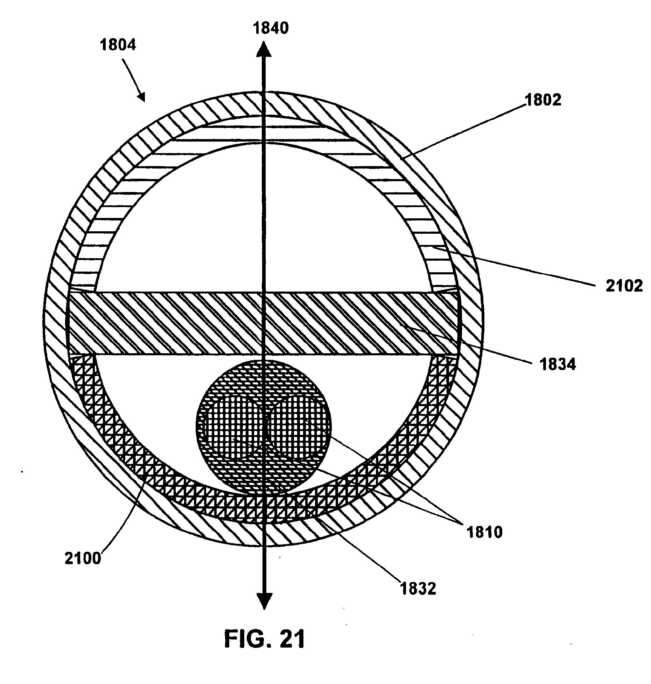

FIG. 21 shows a cross-sectional profile of the distal-heater section 1804 of the catheter 1300. The distal-heater section 1804 is divided into two semicylindrical compartments by the member 1834. In this embodiment the heater 2100 sweeps out an arcuate portion equal to approximately half of the interior diameter of polyamide outer sheath 1804. The opposing arcuate segment of the outer polyamide sheath contains a reflective surface 2102 which tends to insulate the side of the distal-heater section opposite the arcuate heater 2100. Thus heat is generated on only one side of the distal-heater section. This has the advantage of allowing nerves or tissues on the reflective side 2101 of the distal-heater section to be shielded from the heater section 2100.

### Example 6

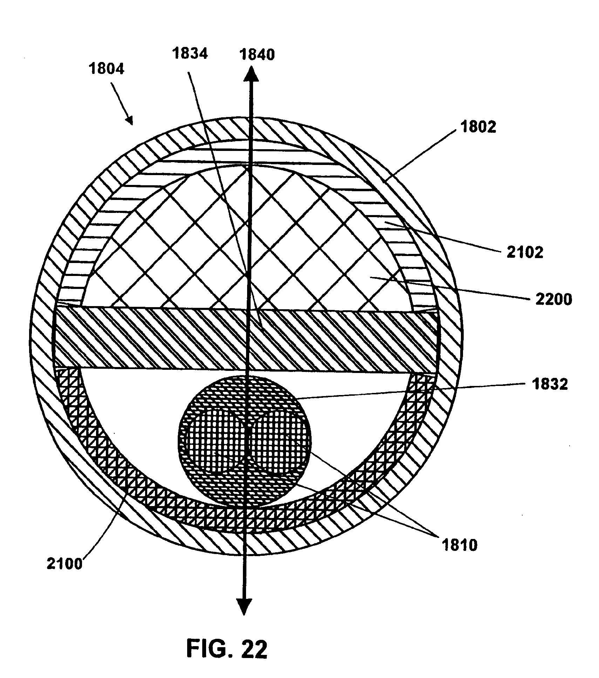

FIG. 22 shows an alternate embodiment of the split-tip distal-heater design discussed above in connection with FIG. 21. Stainless steel core member 1834 divides polyamide outer sheath 1802 into a heated and an unheated portion. The heater portion contains arcuate heater 2100 which sweeps out an arcuate segment of approximately 160° on the inside of polyamide outer sheath 1802. The insulated side of the distal-heater portion 1804 contains both a reflective surface 2102 and/or an insulator 2200 which fills up the void on the insulated half of the distal-heater portion.

### Examples 7-9

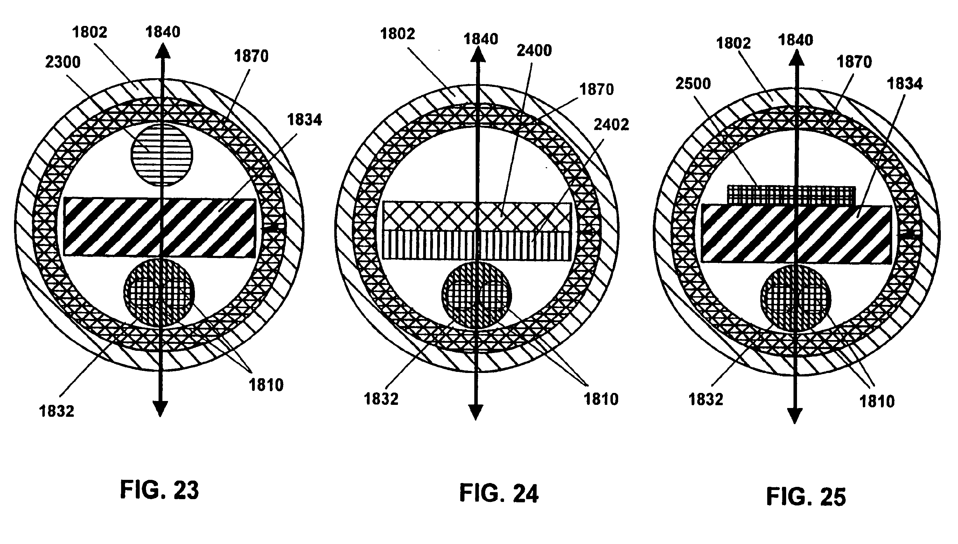

FIGS. 23-25 show alternate embodiments for controlling deflection of the transition 1802 and distal-heater portions 1804 of the catheter 1300 (see FIG. 18). In FIG. 23 heater coil 1870 is positioned within polyamide outer sheath 1802. Bending along axis 1840 is brought about by draw cable 2300 which is fastened to the distal portion of stainless steel core 1834 and which extends along the length of catheter 1300 to the catheter handle 1304 (see FIG. 18). As cable 2300 is drawn out of handle 1304 stainless steel core 1834 is caused to bend in order to relieve the tension created by the cable. Thus in addition to the ability to guide the catheter by rotating the cannula, the catheter can also be guided by retracting cable 2300.

FIG. 24 discloses an alternate embodiment for causing deflection about axis 1840 of the catheter 1300 (see FIG. 18). In this embodiment a bimetallic core member is created by the lamination of dissimilar metals 2400 and 2402 one to another. Each metal has a different coefficient of expansion within the temperature range provided by heater coil 1870. As the heat is delivered to the heater coil, the metal with the higher expansion coefficient induces bending toward the metal with the lesser expansion coefficient along the deflection axis 1840.

FIG. 25 shows an alternate embodiment for generating deflection on axis 1840. In this embodiment a piezoelectric crystal 2500 is laminated to stainless steel core 1834. The piezoelectric crystal is connected electrically to circuitry within handle 1304 (see FIG. 18). The circuitry is capable of generating a voltage which when applied to piezoelectric crystal 2500 causes that crystal to contract or expand with the resultant deflection of stainless steel core 1834 in either direction along axis 1840.

Any of the deflection embodiments discussed above in connection with FIGS. 23-25 provides an extra degree of control for assisting the surgeon to place the distal-heater portion in the desired portion of the spinal disk. This can be useful when, for example, a disk has degenerated sufficiently that the normal guiding action of the interior wall of the spinal disk is no longer present.

### Example 10

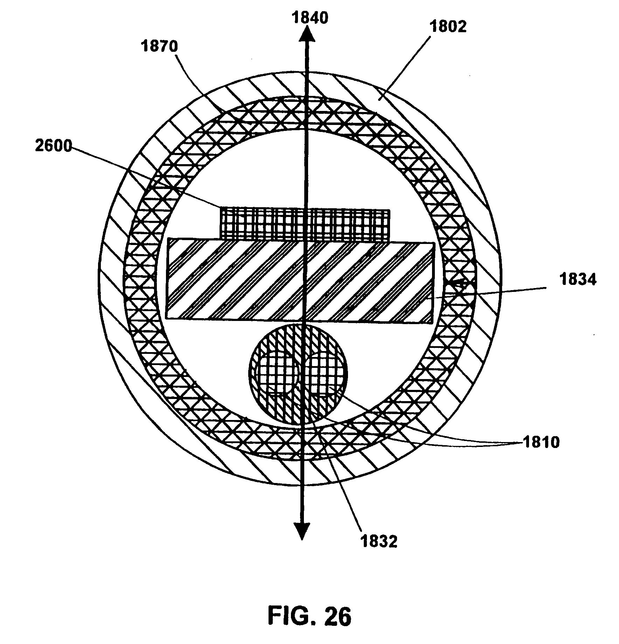

FIG. 26 shows an embodiment similar to that shown in FIG. 25 in which a piezoelectric crystal 2600 is laminated to stainless steel core 1834. In this embodiment voltage may either be delivered to or generated by the piezoelectric crystal 2600. Piezoelectric crystals have the property that when a voltage is applied to them they elongate and/or contract and have the additional property that when they are compressed or stretched they also generate a voltage. Thus a piezoelectric crystal may be used both to deflect as well as to monitor deflection of the distal-heater portion. Thus the surgeon can be provided with a warning signal when a piezoelectric crystal 2600 undergoes compression as a result of the deflection of stainless steel core member 1834. This can be useful in determining the onset of a buckling condition in the distal-heater portion of the catheter 1300 (see FIG. 18).

### Example 11

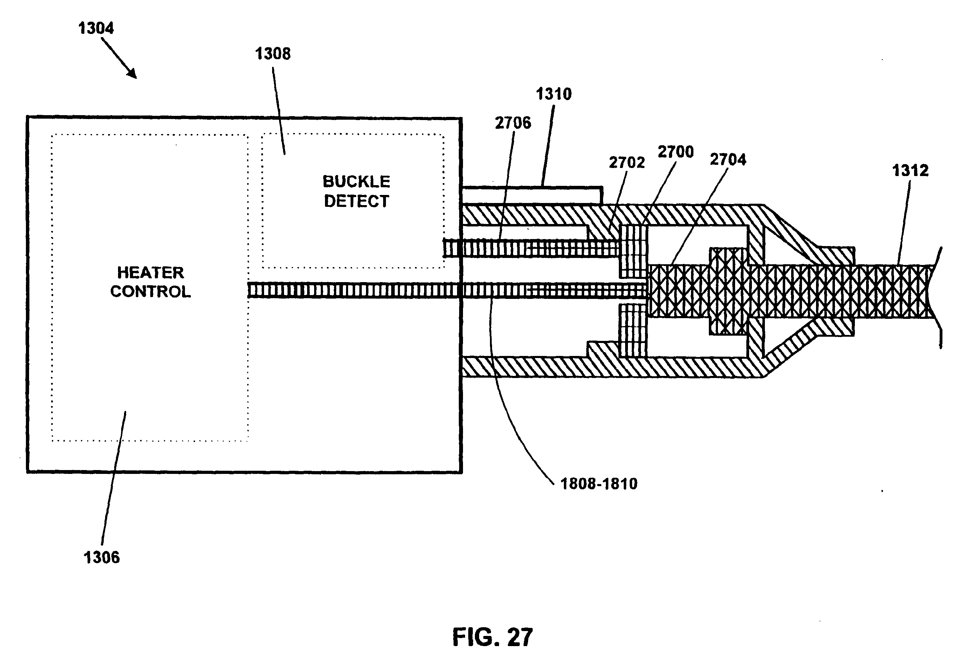

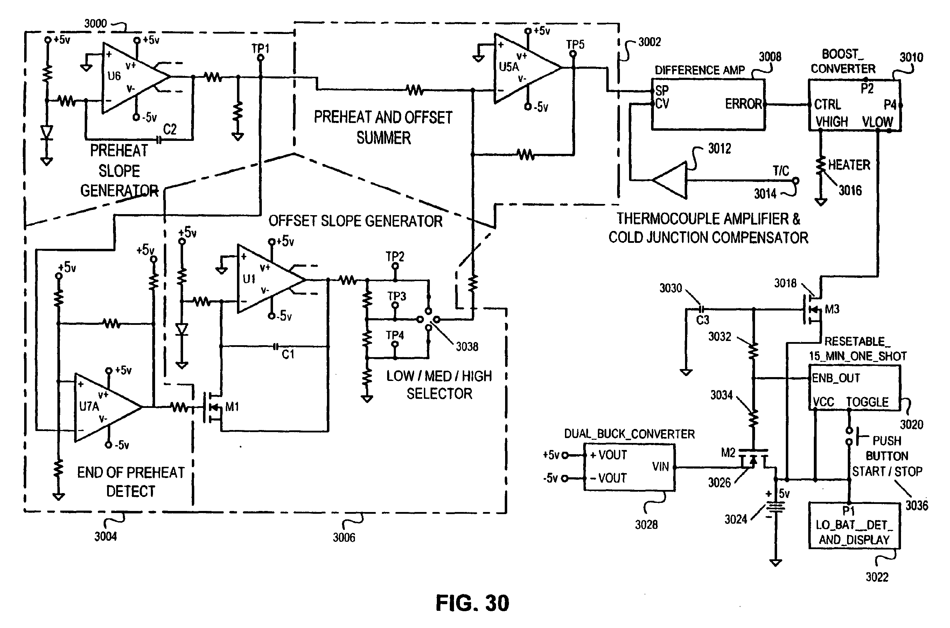

FIG. 27 shows an alternate method for determining the onset of catheter buckling. In this embodiment a piezoelectric crystal 2700 is contained within handle 1304. Specifically the piezoelectric crystal 2700 acts as a thrust bearing between a proximal end of 2704 of catheter stem 1312 and a shoulder 2702 within the handle. The piezoelectric crystal is electrically connected to buckle detection circuitry 1308 within handle 1304. As the compressive force applied by the surgeon on handle 1304 increases to the point where buckling of the catheter is probable, an alarm condition based on the voltage generated by the piezoelectric crystal 2700 can warn the physician that the pressure on the catheter is exceeding acceptable limits. Thus a potentially damaging condition to the patient can be avoided. FIG. 27 also shows a heater generation and control circuit 1306 connected to the thermal couple and heater wires 1808-1810. This circuitry will be described in greater detail in connection with FIGS. 30-35.

### Example 12

FIGS. 28A-B are side and cross-sectional views of a catheter 2800 suitable for material delivery and removal. The catheter includes a handle 2802, dual lumen connectors 2804-2806, stem 2808 and tip 2810. Two openings 2812 and 2814 in the distal portion of the catheter allow for material delivery and/or removal. FIG. 28B is a cross-sectional view of the catheter shown in FIG. 28A taken along lines A-A. A polyimide outer sheath 2850A contains a stainless steel core 1834. The stainless steel core 1834 is held within polyimide outer sheath 2850A by polyimide interior walls 2850B-C. Two lumens 2852-2854 are defined between the polyimide outer sheath 2850A and respectively walls 2850B-C. Lumen 2852 connects opening 2814 to connector 2804. Lumen 2854 connects opening 2812 to connector 2806.

As discussed above, the lumens can be used to deliver biocompatible materials for repairing a fissure or aperture. In an alternate embodiment lumens can be used to deliver heated fluid for therapeutic benefit. In an alternate embodiment lumens 2852 and 2854 can be used to flush out and remove decayed material from the nucleus pulposus. In an alternate embodiment lumens 2852 and 2854 can be used to deliver an antiseptic to the nucleus pulposus.

### Examples 13-14

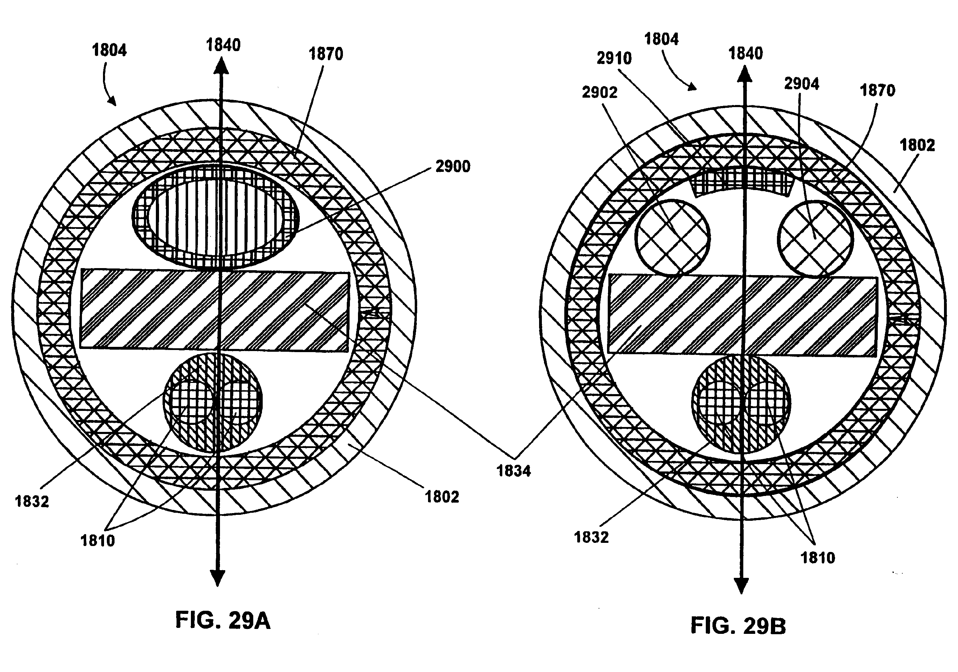

FIGS. 29A-B show alternate embodiments of a catheter which combines one or more lumens with a heater coil. In the embodiment shown in FIG. 29A a heater coil 1870 is contained within an outer polyimide sheath 1802. In the annulus defined between heater coil 1870 and stainless steel core 1834 a lumen 2900 is contained. The combination of a lumen with the heater coil allows a number of desirable features. In an embodiment the lumen can be used to deliver material, to aspirate material or to induce cooling.

In FIG. 29B heater coil 1870 is contained within outer polyimide sheath 1804. Two lumens 2902 and 2904 are contained within the annulus defined by stainless steel core 1834 and heater 1870. These lumens can be used as discussed above in connection with FIG. 29A to aspirate, to deliver and to remove material and to cool a surgical site. Shown additionally in FIG. 29B a piezoelectric crystal 2910 can be laminated either to the interior wall of the heater coil or to the stainless steel core 1834 to monitor bending or to generate deflection.

### Catheter Material Properties

The catheters can have a wide range of physical properties. The compressive strength of the catheter is an important property. The compressive strength of various sections of a catheter representing an embodiment of the invention were determined by preparing a 1 1/8 inch length strip sample of the section of interest with 1/16 of an inch fixed at each end (total length of section that was unconstrained under compression was 1 inch). The samples were compressed at a rate of. 5 inches per minute.

The buckling force of the heater and the transitions at the heater were approximately 1.4 pounds. A good range of buckling force for the heater is 1-2 pounds. A good range of buckling force for an ideal transition is from 1-2 pounds at the heater to 4-5 pounds at proximal shaft. The buckling force of the proximal shaft was approximately 17.5 pounds. A good range of buckling force for the proximal shaft is 10-25 pounds.

The moment of inertia-stiffness of the catheter is another important physical property. To test the moment of inertia-stiffness, a 3 inch length strip was bent in a standard Taber Stiffness Tester on a 10-100 scale. The stiffness of the flexible plane of the heater and transition sections was approximately 21 (Taber stiffness units). A good range of stiffness for the flexible plane of the heater and transition sections is 15-30 (Taber stiffness units). The stiffness of the rigid plane of heater section was approximately 95 (Taber stiffness units). A good range of stiffness for the rigid plane of the heater section is 4-10 times stiffer than the corresponding flexible plane.

Possible materials for the outer sheath of the catheter include polyimide, polyamide, fluropolymer, and silicone. Possible materials for the inertia element (center spine of the device) include stainless steel, Nitinol, and fiber reinforced nylon. Possible materials for the heater coil include nickel chromium alloys (e.g., 80/20 Nichrom), iron/nickel/chromium alloys, stainless steel, and platinum. Possible materials for the marking bands and tip include stainless steel, platinum, and gold. Possible materials for the proximal shaft include stainless steel, aluminum, and fiber reinforced ABS. Possible materials for the introducer cannula include stainless steel. Possible materials for the introducer hub include barium sulfate doped K-resin.

**MATERIAL PROPERTIES TABLE HEATER**

| Material * | Melt Temp **°C** | Resistivity nΩ·m | CTE µm/m°C | TC W/m°K | Spec. Heat J/Kg°K | Tensile ksi | Elongation % | Cost relative |
|---|---|---|---|---|---|---|---|---|
| Aluminum | 600 | 27 | 24 | 247 | 900 | 7-16 | 50-70 | low |
| Antimony, sb | 730 | 370 | 8-11 | 26 | 207 | 2 | | |
| Copper,cu | 1084 | 17 | 16.5 | 398 | 386 | 17-19 | 60 | low+ |
| Gold, au | 1064 | 24 | 14.2 | 318 | 128 | 19 | 30 | high |
| Iron, Fe | 1538 | 970 | 11.7 | 75 | 480 | 26-32 | | low- |
| Nickel | 1453 | 70 | 15 | 83 | 471 | 46 | 30 | low+ |
| Platinum | 1769 | 106 | 9 | 71 | 132 | 18-24 | 30-40 | high+ |
| Silver, Ag | 962 | is | 19 | 428 | 235 | 18 | | med+ |
| Tin, Sn | 232 | 110 | 20 | 63 | 222 | | | low |
| Titanium | 1668 | 420 | 8.4 | 11 | 522 | 35 | 55 | med |
| Tungsten, W | 3410 | 55 | 4.3 | 167 | 140 | 52-59 | 20-50 | med+ |
| 304 SS | | 720 | 16.2 | 15 | 500 | 75-100 | 40-60 | low+ |
| 80Ni-20Cr | | 1125 | 13 | 12.5 | | 100-200 | | med |
| Constantan | | 500 | 15 | 21 | | | | med- |

| | | | | | | | | |
|---|---|---|---|---|---|---|---|---|
| **Note: Impurities and added alloying elements, heat treatment and subsequent working*/*forming techniques have dramatic effects on many of the properties (e.g. 304SS can be cold worked to 350 ksi). All the above materials are assumed to be in the annealed condition.* | | | | | | | | |

In one embodiment of the invention, the heater is made from 80Ni-20Cr wire. It can be advantageous for the heater to be located along one side of the intradiscal section.

### Example 15

FIG. 30 shows an analog circuit for temperature delivery and control. This circuit is suitable for delivery and control of heat in a surgical instrument such as that described and discussed above. The circuit includes a preheat slope generator 3000, a preheat terminator 3004, an offset heat slope generator 3006, a preheat and offset summer 3002, a difference amplifier 3008, a thermocouple 3014, a thermocouple amplifier 3012, a heater 3016, a DC-DC converter 3010, transistors 3018, 3026, resistors 3032 - 3034, capacitor 3030, resetable delay off-timer 3020, start/stop switch 3036, low battery detector 3022, battery 3024, and DC-DC converter 3028.

The pre-heat slope generator 3000 includes an op-amp U6 in an integrating configuration. The preheat termination detector 3004 includes an op-amp U7A wired as a comparator. The offset heat slope generator 3006 includes an op-amp U1 in an integrating configuration. And a single pole three throw switch 3038. The preheat and offset summer 3002 includes an op-amp U5A in a summing configuration.

The output of the preheat slope generator 3000 is connected as an input to both the preheat termination detector 3004 and the preheat and offset summer 3002. The output of the preheat termination detector 3004 is connected to the input of the offset heat slope generator 3006. The output of the offset heat slope generator 3006 is connected to an input of the preheat and offset summer 3002. The output of the preheat and offset summer 3002 is connected to the set point input of the difference amp 3008. The thermocouple 3014 is connected to the input of the thermocouple amplifier 3012. The output of the thermocouple amplifier 3012 is connected to the control input of the difference amplifier 3008. The error output of the difference amp 3008 is connected to the control input of the DC-DC boost converter 3010. The high voltage output of the DC-DC converter 3010 is connected to the heater 3016. The low voltage input of the DC-DC converter 3010 is connected to the drain of transistor 3018. The source of transistor 3018 is connected to the positive terminal of the battery 3024. The positive terminal of the battery 3024 is connected to the input of the low battery detector and display 3022. And to the supply input of the delay off timer 3020. The normally open start/stop switch 3036 connects the positive terminal of the battery 3024 to the toggle input of the delay off timer 3020. The output of the delay off timer 3020 is connected to the gate of transistor 3026 and via an RC delay circuit to the gate of transistor 3018. The source of transistor 3026 is connected to the positive terminal of battery 3024 and the drain of transistor 3026 is connected to the supply input of the DC-DC converter 3028. The outputs of the DC-DC converter 3028 are connected to all the devices within the circuit to supply power to those devices.

In operation, low battery detector and display 3022 continuously monitors and displays the status of battery 3024. When start/stop switch 3036 is activated the output of the resettable delay off timer is toggled from between either an off state or an on state. In the on state the duration of the on condition is fifteen minutes in this embodiment. At the end of fifteen minutes, the on state automatically transitions to an off state. In the on state, the output of the delay off circuit 3020 close circuits first transistor 3026, then transistor 3018. When transistor 3026 is closed circuit, the DC-DC converter 3028 supplies operational power to all the components within the circuit. After the RC delay, transistor 3018 is also close circuited with the result that power from battery 3024 is provided to DC-DC converter 3010. Initially a zero error condition at the control input of the DC-DC converter 3010 results in no power being applied to heater 3016. Thus initially no power is delivered by the heater at the tip of the surgical instruments such as those discussed above.

Upon activation of the DC-DC converter 3028, the preheat slope generator 3000 ramps over the course of one minute its output from a zero voltage to an output of approximately 1.25 volts. The output of the preheat slope generator is supplied as an input to the preheat termination detector 3004. The preheat termination detector 3004 is configured to transition its normally high voltage output to a low voltage condition when its input from the preheat slope generator reaches 1.25 volts. The output of the preheat termination detector 3004 is connected to the gate of transistor M1. The source and drain of transistor M1 can short the input to the output of integrating op-amp U1 which is part of the off set heat slope generator 3006. When the voltage on the gate of transistor M1 is driven low by the output of preheat termination detector 3004, op-amp U1 begins integrating. The output of op-amp U1 ramps from 0 volts to 5 volts over the course of an eleven minute time frame. A series configuration of resistors forming a voltage ladder is connected to the output of op-amp U1. Thus at the end of eleven minutes, voltages of .75, 1.5, and 2.25 volts are available at a corresponding one of each of the 3 input terminals of the 3 pole single throw switch 3038. This switch allows a user to select a low, medium or high temperature level for the heater 3016.

The output of the offset heat slope generator 3006, i.e. the output of the low medium high selector switch 3038, is connected to an input of preheat and offset summer 3002. The other input of the preheat and offset summer is connected to the output of the preheat slope generator 3000. Both of the voltages from the preheat slope generator and the offset heat slope generator are added by the summer 3002. The output of the summer is provided as a target voltage to the set point input of the difference amplifier 3008. The difference amplifier 3008 outputs a error voltage proportional to the difference between the output of amplifier 3012 and the output of the preheat and offset summer 3002. This error condition drives the control input of the DC-DC boost converter 3010. The boost converter, in turn, delivers more or less power to heater 3016 depending on the error condition.

In another preferred embodiment, the output of the boost converter 3010 can be configured to deliver a linear, proportional, integral or derivative of the error condition as an output to the heater. In an alternate embodiment, the boost converter 3010 can supply power to a heater either in the form of a DC current or an RF current. In the latter case, the heater can be configured either in a monopolar arrangement with the corresponding pad or in a bipolar arrangement with more than one tip on the surgical instrument.

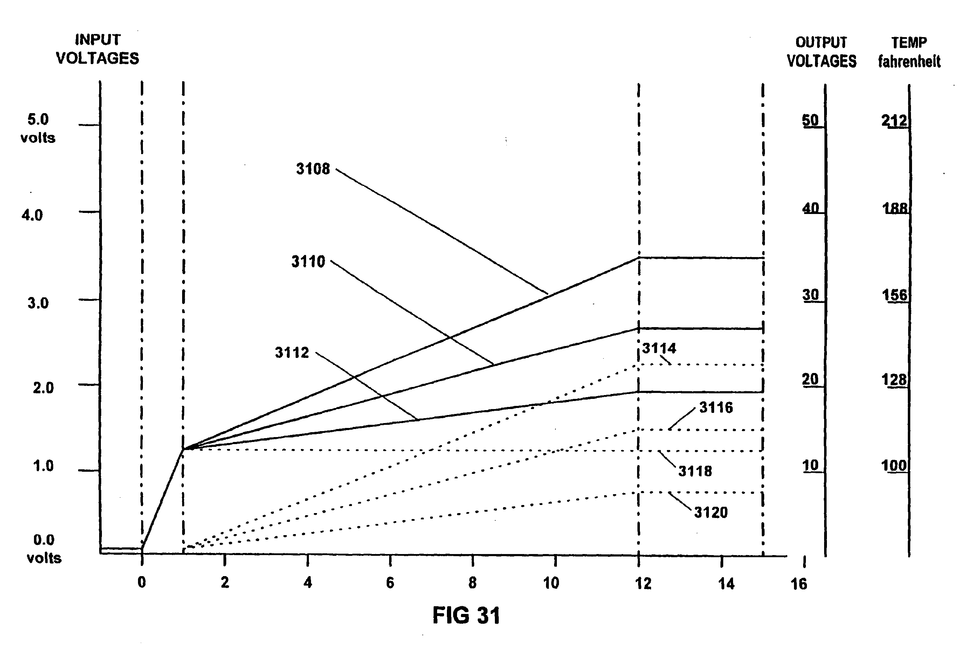

The control circuitry discussed above delivers voltages and temperatures such as those shown in FIG. 31. In FIG. 31, a graph with signal profiles is shown. The graph includes an X axis on which time ranging from T=0 to T=16 minutes is depicted. On the Y axis, 3 input and output voltages and corresponding thermocouple temperatures are shown. Signal 3118 corresponds to the voltage at the output of the preheat slope generator 3000 (see FIG. 30). This output ramps from a zero voltage condition at time T=0 to approximately 1.25 volts at time T=1. This voltage stays at 1.25 volts for the remainder of the operational period, i.e. up until T=15 minutes.

Signals 3114, 3116 and 3120 correspond to the voltages at each one of the 3 positions of switch 3038. These voltages commencing at T=1 minute ramp from a zero voltage level to respectively 2.25, 1.5 and .75 volts at T=12 minutes. From T=12 to T=15 minutes these voltages are fixed, at the above mentioned levels.

The output of the preheat and offset summer 3002 corresponds to one of signal lines 3108 - 3112. These voltages represent the sum of the output of the preheat slope generator 3000 and any one of the outputs of switch 3038.

Thus, at T=0, the output of the preheat and offset summer 3002 is 0 volts. At T=1 minute, the output is 1.25 volts. Depending on the setting of switch 3038, the output of the preheat and offset summer will ramp from 1.25 volts to either 2, 2.75, or 3.5 volts by the time T=12 minutes. These 3 conditions are represented by respectively signal lines 3112, 3110, and 3108. Voltages in either of the 3 switch settings are held constant over the interval from T=12 minutes to T=15 minutes.

At T=15 minutes, the delay off timer 3020 disables first the DC-DC converter 3028, then the DC-DC converter 3010. Thus power is gracefully removed from all components in the circuit except the low battery detector and display. This corresponds to the end of the therapeutic surgical delivery of heat to the patient as discussed above. FIG. 31 also shows alternate Y axis for output voltages and corresponding temperatures in degrees Fahrenheit. The output voltages are the voltages delivered by the DC-DC converter 3010 to the heater 3016 in the resistive heat delivery embodiment. The temperatures in degree Fahrenheit are the temperatures measured by the thermocouple 3014 at each of the output voltages just discussed.

### Example 16

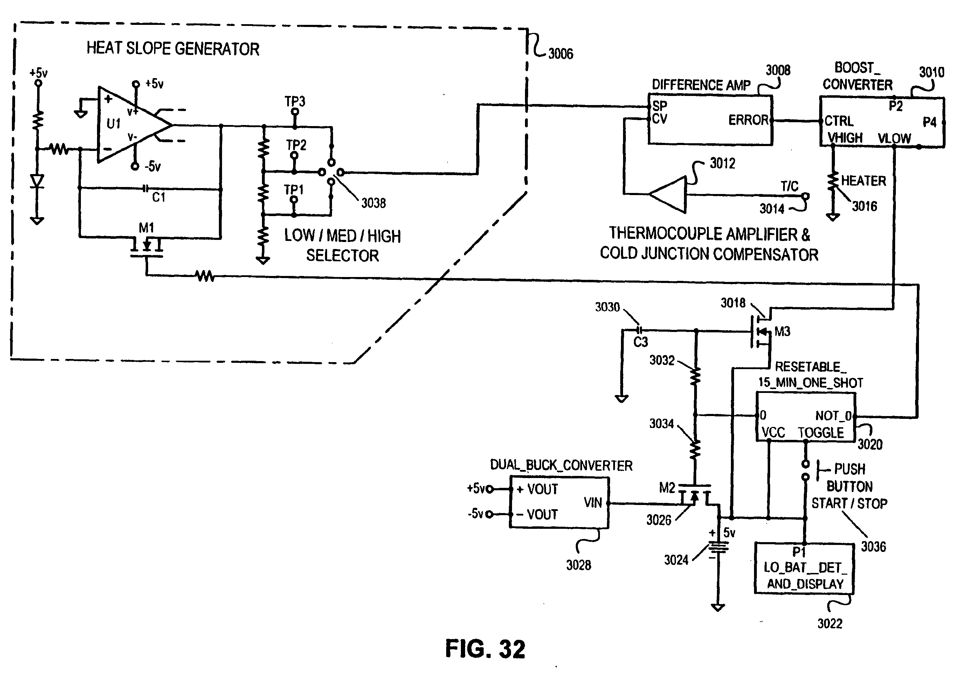

FIG. 32 shows an alternate embodiment of the heat delivery and control device. This embodiment is identical in many respects to that shown in FIG. 30 with the exception of the omission of preheat slope generator 3000, preheat and offset summer 3002 and preheat termination detector 3004. This circuit, therefore, delivers heat without the preheat feature discussed above in connection with FIG. 30.

Heat slope generator 3006 is connected to an input of the difference amp 3008. The thermocouple 3014 is connected to the input of the thermocouple amplifier 3012. The output of the thermocouple amplifier 3012 is connected to the control input of the difference amplifier 3008. The error output of the difference amp 3008 is connected to the control input of the DC-DC boost converter 3010. The high voltage output of the DC-DC converter 3010 is connected to the heater 3016. The low voltage input of the DC-DC converter 3010 is connected to the drain of transistor 3018. The source of transistor 3018 is connected to the positive terminal of the battery 3024. The positive terminal of the battery 3024 is connected to the input of the low battery detector and display 3022. And to the supply input of the delay off timer 3020. The normally open start/stop switch 3036 connects the positive terminal of the battery 3024 to the toggle input of the delay off timer 3020.

An output of the delay off timer 3020 is connected to the gate of transistor M1. Transistor M1 shorts the negative input to the output of integrating op-amp U1 within heat slope generator 3006. When the delay off timer is first initialized via start/stop button 3036, the voltage supplied by resettable delay off timer to the gate of transistor M1 is a low voltage level which open circuits transistor M1 thereby enabling integration to be performed by op-amp U1. The output of the op-amp U1 ramps from a 0 volt to a 3.5 volt state over a 12 minute interval. This output is supplied to one of the 3 inputs of single pole three throw switch 3038. The other 2 inputs of switch 3038 are connected to different nodes of a combination of series resistor which form a voltage divider. Thus 3 different voltage levels are provided from a single output from the op-amp U1 at each of the inputs of switch 3038. In response to a user low medium or high switch setting, a low medium or high set point is delivered to the set point input of difference amp 3008.

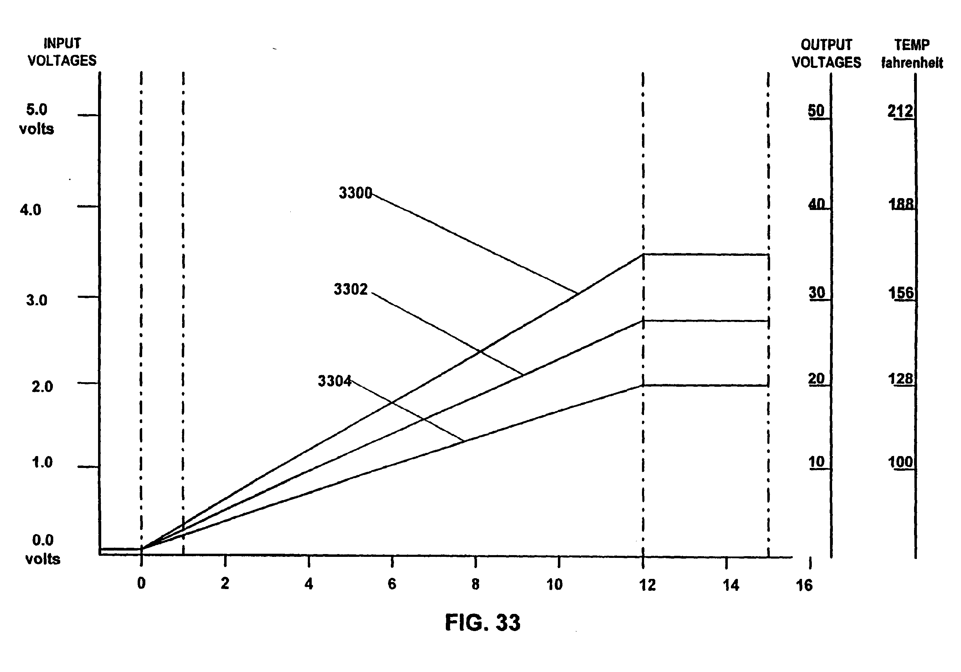

FIG. 33 shows the signal profiles associated with the heat delivery and control circuitry of FIG. 32. At time T=0, the output voltage of the integrating op-amp U1 which is part of the heat slope generator 3006 is 0 volts. When the user initiates heat delivery by start/stop button 3036, power is delivered to the dual buck converter 3028 and after a delay interval, to the DC-DC converter 3010. A low voltage is delivered to the gate of transistor M1 thereby initiating the integration within heat slope generator 3006. Each of the 3 inputs to selector switch 3038 ramps through voltage range shown by signals 3300-3304. At T=12 minutes, signals 3300-3304 have reached a final voltage level of respectively 3.5, 2.75 and 2 volts. These voltage conditions are maintained over the interval from T=12 minutes to T=15 minutes. At T=15 minutes, the resettable delay off timer 3020 shorts the input to the output of the heat slope generator thereby terminating the integration process and shuts down the operation of the circuit. The only remaining active component is the low battery detector and display 3022 and delay off timer.

Shown in FIG. 33 is the output voltage supplied to the heater 3036 by the DC-DC converter 3010. The temperatures in degrees Fahrenheit as measured by thermocouple 3014 are shown with corresponding output voltages.

### Example 17

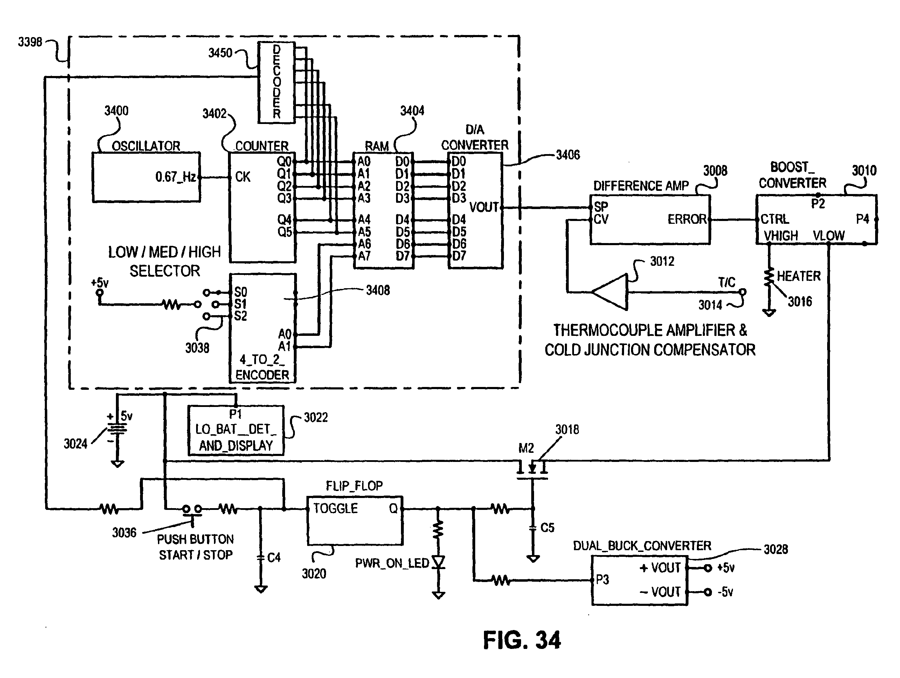

FIG. 34 shows an alternate embodiment of the heat generation and detection circuitry of the current invention. The circuitry disclosed replaces the analog sections 3000-3006 shown in FIG. 30 with a digital section 3398 to achieve the rapid preheating from T=0 to T=1 minute shown in FIG. 31 as well as the more gradual elevation of temperatures from T=1 minute to T=12 minutes shown in FIG. 31.

The circuitry includes a digital section 3398, a difference amp 3008, a DC-DC converter 3010, a thermocouple 3014, a thermocouple amplifier 3012, a heater 3016, a DC-DC converter 3028, a transistor 3018, a delay off timer 3020, a start/stop switch 3036, a low battery detector and display 3022 and a battery 3024. The digital signal generator 3398 includes an oscillator 3400, a counter 3402, a end of treatment decoder 3050, a memory 3404, a digital-2-analog converter 3406, a selector switch 3038, and an encoder 3408.

The oscillator 3400 is connected to the input of up counter 3402. The up counter 3402 has outputs which increment up through a range of memory addresses in response to the input from the oscillator. The counter outputs are connected to the least significant address input bits A0-A5 of the memory 3404 and to the inputs of end or treatment decoder 3050. The output of the end of treatment decoder is connected to the toggle input of flip-flop 3020. The most significant address input bits A6-A7 of memory 3404 are connected to the outputs of encoder 3408. The inputs of encoder 3408 are connected to low medium high selector switch 3038. The data outputs of memory 3404 are connected to the digital inputs ofD/A converter 3406. The output of D/A converter 3406 is connected to the set point input of the difference amp 3008. The control voltage input of the difference amp is connected to the output of thermocouple amplifier 3012. The input of thermocouple amplifier 3012 is connected to thermocouple 3014. The error output of the difference amp 3008 is connected to the control input of the DC-DC boost converter 3010. The high voltage output of the boost converter is connected to the heater 3016. The supply voltage input of the boost converter 3010 is connected to the drain of transistor 3018. The source of transistor 3018 is connected to the positive terminal of the battery 3024. The gate of transistor 3018 is connected through an R/C delay to the output of the delay off timer 3020. The output of the delay off timer is also connected to both a power on LED and an input of the dual buck DC-DC converter 3028. The outputs of the dual buck DC-DC converter are connected to the voltage supply inputs of the various circuit components. The toggle input of the delay off timer 3020 is connected via an R/C delay to the push button start/stop switch 3036. The input of the start/stop switch is connected to the positive terminal of battery 3024. The positive terminal of the battery 3024 is also connected to the input of the low battery detector and display 3022.

RAM 3404 in the example shown contains 8 address input bits coding 256 rows of memory. Each row of memory in the example shown contains one byte of data. Memory is divided into four ranges of 64 rows each. Each of these ranges is selected by the output of encoder 3408. Within any range there are 64 rows. Once the user has selected a specific range, the low medium high selector switch 3038, the oscillator 3400 increments the counter 3402 at approximately 14 second increments across all the addresses within a selected range. Each range of addresses contains a data value corresponding to the desired voltage level at each fourteen second increment within the fifteen minute heat delivery interval. Any temperature profiles can be stored in memory 3404. Memory 3404 may be one-time programmable or reprogrammable. In either case, the voltage values stored in Memory 3404 can follow linear, exponential, integral or derivative profiles. Furthermore, as more rows are added to the memory, a higher resolution of the signal profiles can be provided. The following table shows an example of the contents of memory 3404. Each column represents a different address range. Each row represents a sequential addresses within any of the three ranges. A specific address range is selected by the user via switch 3038 which causes encoder 3408 to select a specific address range on address lines A6-7 of memory 3404. Memory 3404 is in an embodiment programmable non-volatile memory such as EEPROM.

| **SECONDS** | **Address 0-63 TP1 (deg. C)** | **Address 64-127 TP2 (deg. C)** | **Address 128-191 TP3 (deg. C)** |
|---|---|---|---|
| 0 | 40 | 40 | 40 |
| 15 | 40.83 | 41.15 | 41.46 |
| 30 | 41.66 | 42.3 | 42.92 |
| 45 | 42.49 | 43.45 | 44.38 |
| 60 | 43.32 | 44.6 | 45.84 |
| 75 | 44.15 | 45.75 | 47.3 |
| 90 | 44.98 | 46.9 | 48.76 |
| 105 | 45.81 | 48.05 | 50.22 |
| 120 | 46.64 | 49.2 | 51.68 |
| 135 | 47.47 | 50.35 | 53.14 |
| 150 | 48.3 | 51.5 | 54.6 |
| 165 | 49.13 | 52.65 | 56.06 |
| 180 | 49.96 | 53.8 | 57.52 |
| 195 | 50.79 | 54.95 | 58.98 |
| 210 | 51.62 | 56.1 | 60.44 |
| 225 | 52.45 | 57.25 | 61.9 |
| 240 | 53.28 | 58.4 | 63.36 |
| 255 | 54.11 | 59.55 | 64.82 |
| 270 | 54.94 | 60.7 | 66.28 |
| 285 | 55.77 | 61.85 | 67.74 |
| 300 | 56.6 | 63 | 69.2 |
| 315 | 57.43 | 64.15 | 70.66 |
| 330 | 58.26 | 65.3 | 72.12 |
| 345 | 59.09 | 66.45 | 73.58 |
| 360 | 59.92 | 67.6 | 75.04 |
| 375 | 60.75 | 68.75 | 76.5 |
| 390 | 61.58 | 69.9 | 77.96 |
| 405 | 62.41 | 71.05 | 79.42 |
| 420 | 63.24 | 72.2 | 80.88 |
| 435 | 64.07 | 73.35 | 82.34 |
| 450 | 64.9 | 74.5 | 83.8 |
| 465 | 65.73 | 75.65 | 85.26 |
| 480 | 66.56 | 76.8 | 86.72 |
| 495 | 67.39 | 77.95 | 88.18 |
| 510 | 68.22 | 79.1 | 89.64 |
| 525 | 69.05 | 80.25 | 91.1 |
| 540 | 69.88 | 81.4 | 92.56 |
| 555 | 70.71 | 82.55 | 94.02 |
| 570 | 71.54 | 83.7 | 95.48 |
| 585 | 72.37 | 84.85 | 96.94 |
| 600 | 73.2 | 86 | 98.4 |
| 615 | 74.03 | 87.15 | 99.86 |
| 630 | 74.86 | 88.3 | 101.32 |
| 645 | 75.69 | 89.45 | 102.78 |
| 660 | 76.52 | 90.6 | 104.24 |
| 675 | 77.35 | 91.75 | 105.7 |
| 690 | 78.18 | 92.9 | 107.16 |
| 705 | 79.01 | 94.05 | 108.62 |
| 720 | 79.84 | 95.2 | 110.08 |
| 735 | 79. 84 | 95.2 | 110.08 |
| 750 | 79.84 | 95.2 | 110.08 |
| 765 | 79.84 | 95.2 | 110.08 |
| 780 | 79.84 | 95.2 | 110.08 |
| 795 | 79.84 | 95.2 | 110.08 |
| 810 | 79.84 | 95.2 | 110.08 |
| 825 | 79.84 | 95.2 | 110.08 |
| 840 | 79.84 | 95.2 | 110.08 |
| 855 | 79.84 | 95.2 | 110.08 |
| 870 | 79.84 | 95.2 | 110.08 |
| 885 | 79.84 | 95.2 | 110.08 |
| 900 | 79.84 | 95.2 | 110.08 |

Once the user has selected a specific setting on switch 3038, the user then enables start/stop button 3036. After an R/C delay, flip-flop 3020 enables both the DC-DC converters 3028 and 3010. Power is therefore provided to all components in the circuit as indicated by the power on LED shown connected to the output of the flip-flop 3020. The counter 3402 begins up incrementing through the selected address range within memory 3404. The outputs of memory 3404 generate at fourteen second intervals a new digital value to the inputs of the digital-2-analog converter 3406. These outputs are provided to the set point of the difference amp 3008. The difference amp 3008 outputs an error signal proportional to the difference between the set point temperature and the control voltage from the thermocouple 3014. The error signal is provided to the input of the DC-DC boost converter 3010. This causes more or less heat to be delivered to the heater 3016.

At the end of the fifteen minute heat delivery interval, the last address on bits 01-O5 is 11111 binary. When this address is received by end of treatment decoder 3450 provides an output signal which causes flip-flop 3020 to toggle to the off state thereby shutting off the heat delivery to the catheter heater. The flip-flop 3020 removes power from the boost converter 3010 by open circuiting transistor 3018. Additionally, power is removed from the DC-DC converter 3028 thereby ceasing power to supply to all circuit components with the exception of the low battery detector and display 3022.

As discussed above, converter 3010 and heater 3016 may alternately be configured as a R/F control and supply.

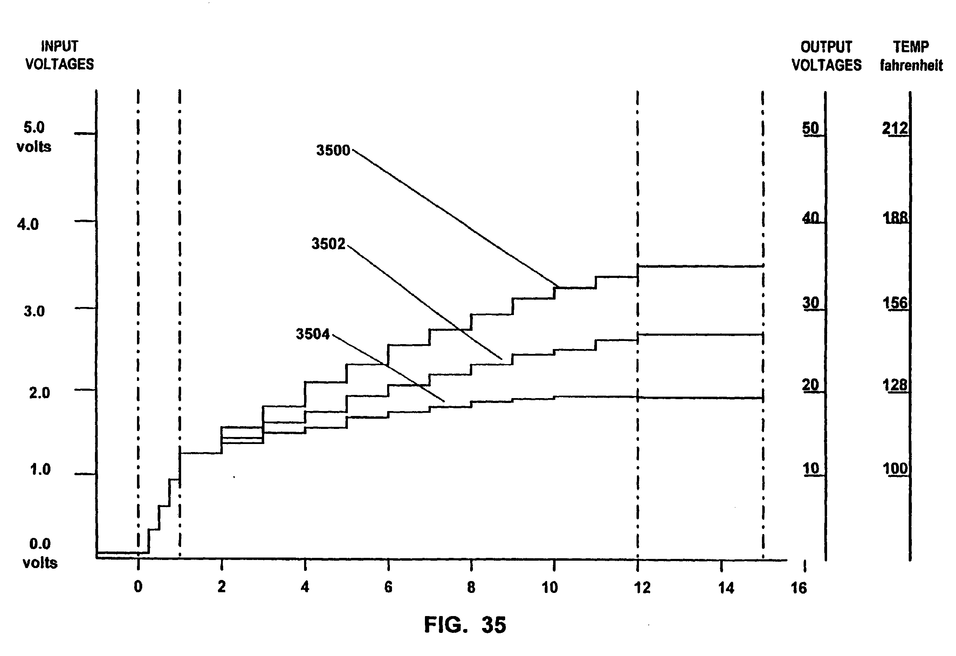

FIG. 35 shows a graph of voltage and temperature versus time for the three voltage ranges discussed above in connection with FIG. 34. The voltages shown by wave forms 3500-3504 correspond to the three possible outputs from T/A converter 3406. Over a preheat interval from 0 to 1 minute, voltages ramp from zero to 1.25 volts in any one of the three selected temperature ranges. In the interval from T=1 to T=12 minutes, each of the three wave forms 3500, 3502 and 3504 settles at a different voltage and respectively 3.5, 2.75 and 2 volts. During the final heat delivery interval from T=12 to T=15 minutes, each of the signals 3500-3504 calls for the voltage level delivered at T=12 minutes to be maintained through T=15 minutes. This allows the specific therapeutic temperature delivery to be maintained. Finally at T=15 minutes, power is removed from the heater and temperatures decay.

Also shown in FIG. 35 are the output voltages from the boost converter 3010 supplied to the resistance heater 3016. The temperature in degree Fahrenheit measured by the thermocouple 3014 at each of these output voltages is also indicated.

All the disclosed embodiments of the invention described herein can be realized and practiced without undue experimentation. Although the best mode of carrying out the invention contemplated by the inventors is disclosed above, practice of the present invention is not limited thereto. The foregoing description of preferred embodiments of the invention has been presented for purposes of illustration and description. It is not intended to be exhaustive or to limit the invention to the precise forms disclosed. Many modifications and variations will be apparent to practitioners skilled in this art. It will be manifest that various additions, modifications and rearrangements of the features of the present invention may be made without deviating from the spirit and scope of the underlying inventive concept. Accordingly, it will be appreciated by those skilled in the art that the invention may be practiced otherwise than as specifically described herein.

For example, the individual components need not be formed in the disclosed shapes, or assembled in the disclosed configuration, but could be provided in virtually any shape, and assembled in virtually any configuration. Further, the individual components need not be fabricated from the disclosed materials, but could be fabricated from virtually any suitable materials. Further, although the device described herein is a physically separate module, it will be manifest that the device may be integrated into the apparatus with which it is associated. Furthermore, all the disclosed elements and features of each disclosed embodiment can be combined with, or substituted for, the disclosed elements and features of every other disclosed embodiment except where such elements or features are mutually exclusive.

All publications, patent applications, and issued patents mentioned in this application are herein incorporated by reference to the same extent as if each individual publication, application, or patent was specifically and individually indicated to be incorporated in its entirety by reference.

It is intended that the appended claims cover all such additions, modifications and rearrangements. The claims are not to be construed as including means-plus-function limitations, unless such limitations are explicitly recited using the term "means" in the claims. Expedient embodiments of the present invention are differentiated by the appended subclaims.

Further aspects of the present invention are defined in the following numbered clauses:
1. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, and iv) having insufficient penetration ability to be advanceable out through said annulus fibrosus under said applied force; and
   at least one energy delivery device located at said intradiscal section for applying energy to said annulus fibrosus, wherein proximity to said nucleus is provided by an introducer having an internal introducer lumen with an opening at a terminus of said introducer.
2. The apparatus of clause 1, wherein at least a portion of said intradiscal section has a differential bending ability in two orthogonal directions at right angles to said longitudinal axis.
3. The apparatus of clause 2, wherein said differential bending ability is controlled by a structural element with different thicknesses in two orthogonal directions at right angles to said longitudinal axis.
4. The apparatus of clause 3, wherein said structural element is maintained in a fixed orientation relative to said catheter by a control element located at said proximal end of said catheter.
5. The apparatus of clause 1, further comprising said introducer, said introducer being capable of puncturing said annulus fibrosus.
6. The apparatus of clause 1, wherein said intradiscal section has a length that is at least one-half of said first diameter of said nucleus pulposus.
7. The apparatus of clause 1, wherein said intradiscal section has a distal portion configured to be non-piercing through said annulus fibrosus.
8. The apparatus of clause 1, wherein said intradiscal section includes a distal portion with a blunt tip.
9. The apparatus of clause 1, wherein said intradiscal section includes a distal portion with a rolling ball tip.
10. The apparatus of clause 1, wherein said intradiscal section has a distal portion that includes a tip that is less rigid than a proximal portion of said intradiscal section.
11. The apparatus of clause 1 , wherein said intradiscal section includes a directionally biased tip portion.
12. The apparatus of clause 1, wherein said energy delivery device includes a thermal energy delivery device.
13. The apparatus of clause 12, wherein said thermal energy delivery device is a resistive heating electrode.
14. The apparatus of clause 12, wherein a thermal energy source is operably attached to said thermal energy delivery device through said catheter.
15. The apparatus of clause 12, wherein said thermal energy delivery device is a microwave probe, and said thermal energy source is a microwave source.
16. The apparatus of clause 12, wherein said thermal energy delivery device is an optical fiber, and said thermal energy source is a laser.
17. The apparatus of clause 12, wherein said thermal energy delivery device is an ultrasound emitter, and said thermal energy source is an ultrasound generator.
18. The apparatus of clause 12, wherein said thermal energy delivery device is a radio frequency electrode, and said thermal energy source is radio frequency generator.
19. The apparatus of clause 1, wherein at least a portion of said intradiscal section of said catheter is actively steerable.
20. The apparatus of clause 1, wherein at least a portion of said catheter is radiographically opaque.
21. The apparatus of clause 1, wherein intradiscal section includes at least one sensor capable of monitoring at least one parameter selected from the group consisting of temperature, power, and voltage, and thereby being capable of controlling heating of said annulus fibrosis.
22. The apparatus of clause 1, wherein said intradiscal section of said catheter includes a wound helical support structure.
23. The apparatus of clause 1, wherein said intradiscal section of said catheter is configured to have at least two radii of curvature when advanced through said nucleus pulposus.
24. The apparatus of clause 1, wherein said catheter includes an irrigation lumen extending from a proximal end of said catheter to said intradiscal section.
25. An apparatus for treating intervertebral disc disease in an intervertebral disc in need thereof, said apparatus comprising:
   a catheter having a distal end, a proximal end and a longitudinal axis, said distal end having an intradiscal section with at least one energy delivery device;
   means for applying a force longitudinally to said proximal end of said catheter which is sufficient to advance said intradiscal section through a nucleus pulposus and around an inner wall of an annulus fibrosus, but which force is insufficient for said intradiscal section to puncture said annulus fibrosus, thereby positioning said energy delivery device at a selected location of said annulus; and
   means for causing said energy delivery device to supply sufficient energy to said selected location to treat said intervertebral disc.
26. The apparatus of clause 25, wherein said means for applying said force includes means for advancing said catheter and twisting said proximal end of said catheter.
27. The apparatus ofclause 25, wherein said means for causing includes means for coupling radio frequency energy from said energy delivery device to said selected location.
28. The apparatus of clause 25, wherein said means for applying said force effectively locates said energy delivery device at a position of said annulus fibrosus selected from the group consisting of posterior lateral, posterior medial, and anterior medial.
29. The apparatus of clause 25, further comprising means for providing an introducer with a proximal end and a distal end and having an introducer lumen with a distal opening at a terminus of said introducer;
   means for inserting said introducer so that said distal end of said introducer is inside said annulus or adjacent to said bulge; and
   means for slidably positioning said catheter in said introducer lumen, wherein said steps of providing an introducer, inserting said introducer, and slidably positioning said catheter are all performed before said step of applying said force.
30. The apparatus of clause 25, wherein said means for causing said energy delivery device to supply sufficient energy includes means for adding energy sufficient to shrink and stiffen a collagen component of said annulus fibrosus.
31. The apparatus of clause 25, wherein said means for providing a catheter includes means for providing a catheter with a lumen capable of delivering or aspirating a material.
32. The apparatus of clause 31, further comprising means for placing a material in said intervertebral disc.
33. The apparatus of clause 32, wherein said means for placing said material includes means for placing in said intervertebral disc at least one member of the group consisting of: irrigation solutions, contrast media, pharmaceutical agents, chemonucleolytic enzymes, hydrogel, osteoinductive substances, chondrocyte-inductive substances, cements, adhesives, collagen, fibrinogen, and thrombin.
34. The apparatus of clause 33, further comprising means for heating said material to seal said material in place.
35. A method of treating intervertebral disc disease in an intervertebral disc in need thereof, said method comprising:
   providing a catheter having a distal end, a proximal end and a longitudinal axis, said distal end having an intradiscal section with at least one energy delivery device;
   applying a force longitudinally to said proximal end of said catheter which is sufficient to advance said intradiscal section through a nucleus pulposus and around an inner wall of an annulus fibrosus, but which force is insufficient for said intradiscal section to puncture said annulus fibrosus, thereby positioning said energy delivery device at a selected location of said annulus; and
   causing said energy delivery device to supply sufficient energy to said selected location to treat said intervertebral disc.
36. The method of clause 35, wherein said step of applying said force includes advancing said catheter and twisting said proximal end of said catheter.
37. The method of clause 35, wherein said step of causing includes coupling radio frequency energy from said energy delivery device to said selected location.
38. The method of clause 35, wherein said step of applying said force effectively locates said energy delivery device at a position of said annulus fibrosus selected from the group consisting of posterior lateral, posterior medial, and anterior medial.
39. The method of clause 35, further comprising providing an introducer with a proximal end and a distal end and having an introducer lumen with a distal opening at a terminus of said introducer,
   inserting said introducer so that said distal end of said introducer is inside said annulus or adjacent to said bulge; and
   slidably positioning said catheter in said introducer lumen, wherein said steps of providing an introducer, inserting said introducer, and slidably positioning said catheter are all performed before said step of applying said force.
40. The method of clause 35, wherein said step of causing said energy delivery device to supply sufficient energy includes adding energy sufficient to shrink and stiffen a collagen component of said annulus fibrosus.
41. The method of clause 35, wherein said steps of applying said force and causing said energy delivery device to supply sufficient energy are repeated at least once.
42. The method of clause 41, wherein said steps of applying said force and causing said energy delivery device to supply sufficient energy are repeated until an entire disc circumference is treated.
43. The method of clause 35, wherein said step of providing a catheter includes providing a catheter with a lumen capable of delivering or aspirating a material.
44 The method of clause 43, further comprising placing a material in said intervertebral disc.
45. The method of clause 44, wherein said step of placing said material includes placing at least one member of the group consisting of: irrigation solutions, contrast media, pharmaceutical agents, chemonucleolytic enzymes, hydrogel, osteoinductive substances, chondrocyte-inductive substances, cements, adhesives, collagen, fibrinogen, and thrombin.
46. The method of clause 45, wherein said step of placing a material is followed by a step of heating said material to seal said material in place.
47. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, iv) having insufficient penetration ability to be advanceable out through said annulus fibrosus under said applied force, and v) having at least a portion with differential bending ability in two orthogonal directions at right angles to the longitudinal axis; and
   at least one energy delivery device located at said intradiscal section for applying energy to said annulus fibrosus, wherein proximity to said nucleus is provided by an introducer having an internal introducer lumen with an opening at a terminus of said introducer,
   wherein the differential bending ability is controlled by a structural element with different thicknesses in the two orthogonal directions.
48. The apparatus of clause 47, further comprising an oval outer sheath that surrounds said portion
49 The apparatus of clause 48, further comprising an oval cannula that surrounds said oval outer sheath and maintains said differential bending ability of said catheter in a fixed orientation relative to said oval cannula.
50. An apparatus for treating intervertebral disc disease in an intervertebral disc in need thereof, said apparatus comprising:
   a catheter having a distal end, a proximal end and a longitudinal axis, said distal end having an intradiscal section i) with at least one energy delivery device and ii) having at least a portion with differential bending ability in two orthogonal directions at right angles to the longitudinal axis;
   means for applying a force longitudinally to said proximal end of said catheter which is sufficient to advance said intradiscal section through a nucleus pulposus and around an inner wall of an annulus fibrosus, but which force is insufficient for said intradiscal section to puncture said annulus fibrosus, thereby positioning said energy delivery device at a selected location of said annulus; and
   means for causing said energy delivery device to supply sufficient energy to said selected location to treat said intervertebral disc,
   wherein the differential bending ability is controlled by a structural element with different thicknesses in the two orthogonal directions.
51. The apparatus of clause 50, further comprising an oval outer sheath that surrounds said portion
52. The apparatus of clause 51, further comprising an oval cannula that surrounds said oval outer sheath and maintains said differential bending ability of said catheter in a fixed orientation relative to said oval cannula.
53. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, and iv) having insufficient penetration ability to be advanceable out through said annulus fibrosus under said applied force; and
   at least one energy delivery device located at said intradiscal section for applying energy to said annulus fibrosus, wherein proximity to said nucleus is provided by an introducer having an internal introducer lumen with an opening at a terminus of said introducer,
   wherein said intradiscal section is separated into a heated portion and an unheated portion and said at least one energy delivery device is located in said heated portion.
54. The apparatus of clause 53, further comprising a reflective surface in said unheated portion.
55. The apparatus of clause 53, wherein said at least one energy delivery device includes an arcuate heater.
56. The apparatus of clause 53, wherein said unheated portion includes insulation to reduce that amount of heat delivered by said unheated portion.
57. An apparatus for treating disease, comprising:
   a catheter having a distal end, a proximal end, and a longitudinal axis, said catheter having a section at said distal end of said catheter, said section including an active deflector adapted to deflect said distal end.
58 The apparatus of clause 57, wherein said active deflector includes a draw cable.
59. The apparatus of clause 57, wherein said active deflector includes a piezoelectric circuit.
60. The apparatus of clause 59, wherein said piezoelectric circuit operates in a passive mode as a buckling detector.
61. The apparatus of clause 57, wherein said active deflector includes a differential thermal expansion assembly.
62. The apparatus of clause 57, wherein said active deflector includes a shape memory alloy.
63. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, iv) having insufficient penetration ability to be advanceable out through said annulus fibrosus under said applied force, and v) including an active deflector adapted to deflect said distal end; and
   at least one energy delivery device located at said intradiscal section for applying energy to said annulus fibrosus, wherein proximity to said nucleus is provided by an introducer having an internal introducer lumen with an opening at a terminus of said introducer.
64. The apparatus of clause 63, wherein said active deflector includes a draw cable.
65. The apparatus of clause 63, wherein said active deflector includes a piezoelectric circuit.
66. The apparatus of clause 65, wherein said piezoelectric circuit operates in a passive mode as a buckling detector.
67. The apparatus of clause 63, wherein said active deflector includes a differential thermal expansion assembly.
68. The apparatus of clause 63, wherein said active deflector includes a shape memory alloy.
69. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, and iv) having insufficient penetration ability to be advanceable out through said annulus fibrosus under said applied force;
   at least one energy delivery device located at said intradiscal section for applying energy to said annulus fibrosus, wherein proximity to said nucleus is provided by an introducer having an internal introducer lumen with an opening at a terminus of said introducer; and
   at least one lumen located within said catheter.
70. The apparatus of clause 69, wherein said at least one lumen includes a first lumen located within said catheter, and a second lumen located within said catheter.
71. The apparatus of clause 69, further comprising an energy delivery device located in said intradiscal section.
72. The apparatus of clause 69, further comprising a thermocouple located in said intradiscal section.
73. The apparatus of clause 69, further comprising a piezoelectric circuit located in said intradiscal section.
74. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, and iv) having insufficient penetration ability to be advanceable out through said annulus fibrosus under said applied force;
   at least one energy delivery device located at said intradiscal section for applying energy to said annulus fibrosus, wherein proximity to said nucleus is provided by an introducer having an internal introducer lumen with an opening at a terminus of said introducer; and
   a buckling detection assembly located at said proximal end.
75. The apparatus of clause 74, wherein said buckling detection assembly includes a piezoelectric crystal that is contained within a handle, wherein the piezoelectric crystal acts as a thrust bearing between said proximal end of said catheter and a shoulder located within said handle.
76. An externally guidable intervertebral disc apparatus for manipulation of disc tissue at a selected location of an intervertebral disc, the apparatus comprising:
   a catheter having a distal end, a proximal end, and a longitudinal axis, the catheter having an intradiscal section at the distal end of the catheter, the intradiscal section being extendible into the disc and having sufficient rigidity to be advanceable through a nucleus pulposus and around an inner wall of an annulus fibrosus under a force applied longitudinally to the proximal end, having sufficient flexibility in a direction of a disc plane to be compliant with the inner wall, and having insufficient penetration ability to be advanceable out through the annulus fibrosus under the applied force; and
   at least one functional element located in said intradiscal section to perform at least one function from the following:
      adding material at said selected location of said intervertebral disc, and
      removing material at said selected location of said intervertebral disc.
77. The apparatus of clause 76, wherein at least a portion of the intradiscal section further has differential bending ability in two orthogonal directions at right angles to the longitudinal axis.
78. The apparatus of clause 77, wherein the differential bending ability is controlled by a structural element with different thicknesses in the two orthogonal directions.
79. The apparatus of clause 76, wherein said at least one functional element includes a lumen.
80. The apparatus of clause 79, wherein the lumen is an irrigation lumen extending from a proximal end of the catheter to the intradiscal section where it administers fluids and fluid-borne materials.
81. A method of manipulating a disc tissue at a selected location of an intervertebral disc, the disc having a nucleus pulposus, an annulus fibrosus, and an inner wall of the annulus fibrosus, the nucleus pulposus having a diameter in a disc plane between opposing sections of the inner wall, the method comprising:
   providing a catheter having a functional element, a distal end, a proximal end and a longitudinal axis;
   applying a sufficient force to advance the catheter through the nucleus pulposus and around the inner wall of the annulus, which force is insufficient to puncture the annulus fibrosus;
   positioning the functional element at the selected location of the disc by advancing or retracting the catheter and optionally twisting the proximal end of the catheter; and manipulating the disc tissue at the selected location of the disc via the functional element.
82. The method of clause 81, wherein manipulating the disc tissue comprises adding or removing a material.
83. The method of clause 81, wherein the selected location is along the inner wall of the annulus fibrosus.
84. The method of clause 81, wherein the selected location is along a posterior medial inner, posterior lateral, anterior lateral or anterior medial wall of the annulus fibrosus or a combination thereof.
85. The method of clause 81, wherein the step of providing the catheter further comprises providing a catheter with multiple thermal delivery elements and the intradiscal section is positioned to conform to and denervate at least a portion of the circumference of the inner wall the annulus fibrosus.
86. An externally guidable intervertebral disc treatment apparatus for treating disease in an intervertebral disc in need thereof, said intervertebral disc having a nucleus pulposus, an annulus fibrosus, and an inner wall of said annulus fibrosus, said nucleus pulposus having a first diameter in a disc plane between opposing sections of said inner wall, said externally guidable intervertebral disc treatment apparatus comprising:
   a catheter having a distal end, a proximal end, and a longitudinal axis, said catheter having an intradiscal section at said distal end of said catheter, said intradiscal section i) being extendible into said disc and having sufficient rigidity to be advanceable through said nucleus pulposus and around said inner wall of said annulus fibrosus under a force applied longitudinally to said proximal end, ii) having sufficient flexibility to be compliant with said inner wall, iii) having sufficient flexural strength in other directions to follow said inner wall, and iv) having insufficient penetration ability to be advanceable out through said annulus fibrosus under said applied force;
   at least one energy delivery device located at said intradiscal section for applying energy to said annulus fibrosus, wherein proximity to said nucleus is provided by an introducer having an internal introducer lumen with an opening at a terminus of said introducer; and
   a circuit for supplying energy and controlling temperature connected to said at least on energy delivery device.
87. An apparatus for treating intervertebral disc disease in an intervertebral disc in need thereof, said apparatus comprising:
   a catheter having a distal end, a proximal end and a longitudinal axis, said distal end having an intradiscal section with at least one energy delivery device;
   means for applying a force longitudinally to said proximal end of said catheter which is sufficient to advance said intradiscal section through a nucleus pulposus and around an inner wall of an annulus fibrosus, but which force is insufficient for said intradiscal section to puncture said annulus fibrosus, thereby positioning said energy delivery device at a selected location of said annulus;
   means for causing said energy delivery device to supply sufficient energy to said selected location to treat said intervertebral disc; and
   a circuit for supplying energy and controlling temperature connected to said at least on energy delivery device.
88. A method of treating intervertebral disc disease in an intervertebral disc in need thereof, said method comprising:
   providing a catheter having a distal end, a proximal end and a longitudinal axis, said distal end having an intradiscal section with at least one energy delivery device;
   applying a force longitudinally to said proximal end of said catheter which is sufficient to advance said intradiscal section through a nucleus pulposus and around an inner wall of an annulus fibrosus, but which force is insufficient for said intradiscal section to puncture said annulus fibrosus, thereby positioning said energy delivery device at a selected location of said annulus;
   causing said energy delivery device to supply sufficient energy to said selected location to treat said intervertebral disc,
   wherein the step of causing includes:
   supplying energy to said energy delivery device; and
   controlling temperature connected to said at least on energy delivery device.
89. An externally guidable surgical device, said externally guidable surgical device comprising:
   a catheter having a distal end, a proximal end, and a longitudinal axis, said catheter having a navigation section at said distal end of said catheter, said navigation section including an active deflector adapted to deflect said distal end.
90. The apparatus of clause 89, wherein said active deflector includes a draw cable.
91. The apparatus of clause 89, wherein said active deflector includes a piezoelectric circuit.
92. The apparatus of clause 91, wherein said piezoelectric circuit operates in a passive mode as a buckling detector.
93. The apparatus of clause 89, wherein said active deflector includes a differential thermal expansion assembly.
94. The apparatus of clause 89, wherein said active deflector includes a shape memory alloy.
95. An energy generator and controller for delivering energy to a surgical site, said energy generator and controller comprising;
   an energy delivery unit to deliver an energy to the surgical site;
   a heat slope generator to generate a target energy level as a function of time;
   a detector to monitor an energy level at the surgical site;
   a difference detector to detect a difference between the target energy level and the monitored energy level and to alter the energy to the surgical site responsive to the detected difference.
96. The energy generator and controller of clause 95, wherein said heat slope generator includes:
   an address incrementer to output each address within a selected range of addresses;
   a memory containing address inputs and data outputs and a set of data entries collectively corresponding to the target energy levels as a function of time, and each member of said set of data entries incrementally selectable by an address output by said address incrementer to said address inputs of said memory, and said data outputs of said memory connected to a target energy level input of said difference detector to generate the target energy level as a function of time.

## Claims

1. An externally guidable intervertebral disc apparatus for manipulation of disc tissue at a selected location of an intervertebral disc, the apparatus comprising;
a catheter having a distal end, a proximal end, and a longitudinal axis, the catheter having an intradiscal section at the distal end of the catheter, the intradiscal section being extendible into the disc and having sufficient rigidity to be advanceable through a nucleus pulposus and around an inner wall of an annulus fibrosus under a force applied longitudinally to the proximal end, having sufficient flexibility in a direction of a disc plane to be compliant with the inner wall, and having insufficient penetration ability to be advanceable out through the annulus fibrosus under the applied force; and
at least one functional element located in said intradiscal section to perform at least one function from the following:
adding material at said selected location of said intervertebral disc, and removing material at said selected location of said intervertebral disc.

2. The apparatus of claim 1, wherein at least a portion of the intradiscal section further has differential bending ability in two orthogonal directions at right angles to the longitudinal axis.

3. The apparatus of claim 2, wherein the differential bending ability is controlled by a structural element with different thicknesses in the two orthogonal directions.

4. The apparatus of claim 1, wherein said at least one functional element includes a lumen.

5. The apparatus of claim 4, wherein the lumen is an irrigation lumen extending from a proximal end of the catheter to the intradiscal section where it administers fluids and fluid-borne materials.
